# EUROPEAN PATENT APPLICATION

(11) **EP 4 442 705 A1**
(43) Date of publication of application: **09.10.2024**
(21) Application number: 23166403.8
(22) Date of filing: 03.04.2023
(51) Int. Cl.: C07K 16/18

(54) **DOSAGE**

(71) Applicant: Citryll B.V., 5349 AB Oss (NL)
(72) Inventor: The designation of the inventor has not yet been filed
(74) Representative: J A Kemp LLP

(57) **Abstract**

The invention provides antibodies or binding fragments thereof directed against citrulline-containing epitopes for use in treating or preventing diseases associated with extracellular trap release from cells, such as Neutrophil Extracellular Trap (NET)-associated pathologies (NET associated pathology) or Eosinophil Extracellular Trap (EET) -associated pathologies (EET-associated pathology), wherein the methods comprising administering at least one dose of the antibody at a specific concentration. The invention also provides the methods themselves. The NET-associated pathologies include systemic lupus erythematosus (SLE), lupus, sepsis, vasculitis, inflammatory arthritis, rheumatoid arthritis and osteoarthritis, psoriasis, Alzheimer's disease, autoimmune hepatitis, juvenile idiopathic arthritis, myositis (polymyositis and dermatomyositis), Sjögren's disease, Anti-phospholipid Syndrome, Bechet's disease, spondylitis, spondyloarthropathy, multiple system atrophy, Parkinson's disease, Lewy body dementia, asthma, allergic rhinovirus exacerbated asthma, allergic asthma, acute respiratory distress syndrome, cystic fibrosis, fibrosis and idiopathic pulmonary fibrosis, heart failure, atherosclerosis, dry eye disease, uveitis, nongranulomatous uveitis, granulomatous uveitis, dermatitis, atopic dermatitis, COPD, bronchitis, or other NET-associated pathologies such as wound healing in diabetes, cancer, cancer metastasis, and transplant organ health *in vivo* or *ex vivo.* The invention also provides pharmaceutical compositions and methods for treating or preventing NET-associated pathologies such as SLE, lupus, sepsis, vasculitis, inflammatory arthritis, rheumatoid arthritis and osteoarthritis, psoriasis, Alzheimer's disease, autoimmune hepatitis, juvenile idiopathic arthritis, myositis (polymyositis and dermatomyositis), Sjögren's disease, Anti-phospholipid Syndrome, Bechet's disease, spondylitis, spondyloarthropathy, multiple system atrophy, Parkinson's disease, Lewy body dementia asthma, allergic rhinovirus exacerbated asthma, allergic asthma, acute respiratory distress syndrome, cystic fibrosis, fibrosis and idiopathic pulmonary fibrosis, heart failure, atherosclerosis, dry eye disease, uveitis, nongranulomatous uveitis, granulomatous uveitis, dermatitis, atopic dermatitis, COPD, bronchitis, thrombotic diseases, cardiovascular diseases or other NET-associated pathologies such as wound healing in diabetes, cancer, cancer metastasis, and transplant organ health *in vivo* or *ex vivo.* The EET-associated pathologies include an eosinophilic disease or condition, such as eosinophilic disease or condition of the skin; a respiratory eosinophilic disease or condition; a gastro-intestinal eosinophilic disease or condition; an allergic disease or condition; or a helminth, fungal, viral, or bacterial infection.

## Description

### Field of the Invention

The present invention relates to antibodies or binding fragments thereof directed against citrulline-containing epitopes for use in methods of treating or preventing diseases associated with extracellular trap release from cells, such as Neutrophil Extracellular Trap (NET)-associated pathologies or Eosinophil Extracellular Trap (EET) -associated pathologies, and in particular to the doses of the antibodies or binding fragments thereof to be employed. The present invention further relates to methods of treating or preventing of Neutrophil Extracellular Trap (NET)-associated pathologies or Eosinophil Extracellular Trap (EET) -associated pathologies comprising administering to a subject in need thereof antibodies or binding fragments thereof directed against citrulline-containing epitopes at particular dosages.

### Background of the invention

Whilst the inflammatory process plays a key role in the normal response of the body to infection, in inflammatory disorders the inflammatory process forms part of the underlying pathogenesis of the disorder, for example because the immune system is triggered against the body's own tissues or because the nature of the immune response against a pathogen is too great and causes damage to the body. Given the large number of disorders inflammation plays a role in, it represents a substantial problem in the healthcare industry. Inflammation is often subdivided into acute and chronic inflammation. Chronic inflammation is considered to be inflammation of a prolonged duration (weeks or months) in which active inflammation, tissue destruction and attempts at healing are proceeding simultaneously. Although chronic inflammation can follow an acute inflammatory episode, it can also begin as an insidious process that progresses with time, for example, as a result of a persistent infection (e.g., tuberculosis, syphilis, fungal infection) that causes a delayed hypersensitivity reaction, prolonged exposure to endogenous (e.g., elevated plasma lipids) or exogenous (e.g., silica, asbestos, cigarette tar, surgical sutures) toxins, or autoimmune reactions against the body's own tissues (e.g., rheumatoid arthritis, systemic lupus erythematosus, vasculitis, multiple sclerosis, psoriasis).

Inflammatory disorders include those where the focus of the research has perhaps been on other aspects of the disorder. For example, one such condition is Parkinson's disorder (PD) where much research has focused on the formation of neurofibrillary tangles of tau protein. The underlying pathogenesis of PD though also involves inflammation which is thought responsible for the underlying damage to the central nervous system (CNS), with neutrophils considered to play a role in that inflammation.

One consequence of inflammation is the formation of Neutrophil Extracellular Traps (NETs). NETs are also known to cause inflammation. NETs are structures comprising DNA and histones that are produced by neutrophils as part of the host defense mechanism against pathogens. They can trap and kill various bacterial, fungal, viral and protozoal pathogens, and their release is one of the first lines of defense against pathogens. Following activation by microorganisms or cytokines, histones become hypercitrullinated and the neutrophil nucleus undergoes a process of chromatin decondensation that leads to the formation of NETs by NETosis, a form of neutrophil cell death.

NETs play a pathological role in a variety of diseases, for example by causing aberrant inflammation. Thus, NETs are involved in the pathology of a variety of inflammatory conditions, such as systemic lupus erythematosus (SLE), lupus, sepsis, vasculitis, inflammatory arthritis, rheumatoid arthritis and osteoarthritis, psoriasis, hidradenitis suppurativa, Alzheimer's disease, autoimmune hepatitis, juvenile idiopathic arthritis, myositis (polymyositis and dermatomyositis), Sjögren's disease, Bechet's disease, spondylitis, spondyloarthropathy, multiple system atrophy, Parkinson's disease, Lewy body dementia asthma, allergic rhinovirus exacerbated asthma, COPD, Acute respiratory distress syndrome, cystic fibrosis, idiopathic pulmonary fibrosis, heart failure and atherosclerosis. For example, NETs can cause autoantigen exposure to the extracellular space and the subsequent production of pathological autoantibodies by the subject. Furthermore, NETs and NET remnants harbor toxic histones, which induce vascular damage and subsequent organ damage and failure. Thus, in such diseases, interfering with NET formation, and inducing clearance of NETs and NET remnants from circulation and tissues, would have therapeutic benefits. Neutrophils are also increasingly being recognized as an important element in tumour progression. They have been shown to exert important effects at nearly every stage of tumour progression with a number of studies demonstrating that their presence is critical to tumour development. Studies have also implicated NETs as facilitators of tumour progression and metastasis. It has also been shown that neutrophils, through the generation of NETs, provide a scaffold and a stimulus for platelet adhesion, thrombus formation and coagulation in tumours.

NETs have also been implicated in reducing organ health after transplant. NETs contribute to primary graft dysfunction, contributing to early mortality after lung transplantation. It has been shown that NETs play a pathogenic role in solid organ transplantation.

A promising approach for the treatment of inflammatory diseases by targeting the production of NETs is the use of antibodies that bind to citrullinated epitopes on the amino terminus of histones 2A and/or histone 4. These antibodies can be used to treat diseases or pathologies associated with citrullination, such as NET-associated pathologies and inflammatory conditions. Antibodies that bind to citrullinated epitopes on deiminated human histone 2A and histone 4 are described in WO2009147201, WO2011070172, WO2016092082 and in particular in WO 2020/038963.

Eosinophils are a form of circulating leukocyte, typically representing about 1 to 3% of white blood cells (WBCs) in a healthy human. They have a wide variety of roles in homeostasis and various diseases including allergy and infection. It has been identified that a particular mechanism of active cytolytic eosinophil cell death releases eosinophil extracellular traps (EETs) and total cellular contents. This is referred to as eosinophil extracellular trap cell death (EETosis). It has also been shown that Charcot-Leyden crystals (a classical pathological marker of eosinophilic inflammation) is associated with EETosis, and the presence of EETosis and EETs has been reported in multiple diseases.

Antibodies that bind to citrullinated epitopes on the amino terminus of histones 2A and/or histone 4 and are able to inhibit EETosis are disclosed in WO2022233931. WO2022233931 discloses methods for the treatment or prevention of an EET-associated pathology using said antibodies. Such pathologies may include: an eosinophilic disease of the skin; a respiratory eosinophilic disease; a gastro-intestinal eosinophilic disease; an allergic disease; or a helminth, fungal, viral, or bacterial infection. In addition, arteriosclerosis may also be treated or prevented. In another embodiment, vasculitis may also be treated.

There is a need for further optimisation of treatment of the diseases described above with such antibodies.

### Summary of the invention

The present inventors have identified particularly effective dosages of antibodies or binding fragments that specifically bind to a citrullinated epitope on deiminated human histone 2A and/or histone 4 for use in a method of treating or preventing diseases associated with extracellular trap release from cells, such as Neutrophil Extracellular Trap (NET)-associated pathologies or Eosinophil Extracellular Trap (EET) -associated pathologies in a subject in need thereof. The dosage of antibody or binding fragment is in the range of 0.01 mg/kg to 0.6 mg/kg. In one particularly preferred embodiment the dosages provided results in not just a desired impact on NETs and/or EETs, but also particular cytokine levels which differ from when higher dosages of antibody or binding fragment are employed. For example, in one embodiment the dosages may result in increased IL-10 release in the subject. In another embodiment, the dosage result in a reduced level of at least one of calprotectin, P-selectin, CXCL10, TNF-alpha, and VCAM in the subject.

Accordingly, the present invention provides an antibody or binding fragment thereof that specifically binds to a citrullinated epitope on deiminated human histone 2A and/or histone 4 for use in a method of treating or preventing a disease associated with extracellular trap release from cells, such as a Neutrophil Extracellular Trap (NET)-associated pathology or an Eosinophil Extracellular Trap (EET) -associated pathology in a subject in need thereof, wherein the antibody or binding fragment thereof is administered at a dosage of about 0.01 mg/kg to about 0.6 mg/kg.

The present invention also provides a method for treating a Neutrophil Extracellular Trap associated pathology (a NET-associated pathology) in a subject in need thereof, the method comprising administering an antibody or binding fragment thereof that specifically binds to a citrullinated epitope on deiminated human histone 2A and/or histone 4 to the subject, wherein the antibody or binding fragment thereof is administered at a dosage of about 0.01 mg/kg to about 0.6 mg/kg.
The present invention also provides a method for treating an Eosinophil Extracellular Trap (EET) -associated pathology (an EET-associated pathology) in a subject in need thereof, the method comprising administering an antibody or binding fragment thereof that specifically binds to a citrullinated epitope on deiminated human histone 2A and/or histone 4 to the subject, wherein the antibody or binding fragment thereof is administered at a dosage of about 0.01 mg/kg to about 0.6 mg/kg.

### Brief Description of the Sequence Listing and Nomenclature

### Antibody nomenclature

CDR = complementarity-determining region.
VH = heavy chain variable domain.
VL = light chain variable domain.
CH = heavy chain constant domain.
CL = light chain constant domain.
msVH22.101 = mouse VH of therapeutic antibody.
msVL22.101 = mouse VL of therapeutic antibody.
hVH22.101x = humanized VH of therapeutic antibody, 'x' refers to the heavy chain.
hVL22. 101y = humanized VL of therapeutic antibody, 'y' refers to the light chain.
hVH22.101(HC)x = optimized humanized VH of therapeutic antibody, '(HC)x' refers to the heavy chain.
hVL22.101(LC)y = optimized humanized VL of therapeutic antibody, '(LC)y' refers to the light chain.
hMQ22.101x/y = humanized therapeutic antibody, 'x' refers to the heavy chain, 'y' refers to the light chain.
hMQ22.101(HC)x/(LC)y = optimized humanized therapeutic antibody of the invention, '(HC)x' refers to the heavy chain, '(LC)y' refers to the light chain.

| **SEQ ID NO** | **Protein** | **Name** |
|---|---|---|
| 1 | protein | CDR1 of msVH22.101 and hVH22.101(HC)x |
| 2 | protein | CDR2 of msVH22.101 and hVH22.101(HC)x |
| 3 | protein | CDR3 of msVH22.101 and hVH22.101(HC)x |
| 4 | protein | CDR2 of msVL22.101 and hVL22.101(LC)y |
| 5 | protein | CDR3 of msVL22.101 and hVL22.101(LC)y |
| 6 | protein | CDR1 of hVL22.101LC17 |
| 7 | protein | CDR1 of hVL22.101LC21 |
| 8 | protein | CDR1 of hVL22.101LC27 |
| 9 | protein | CDR1 of hVL22.101LC41 |
| 10 | protein | CDR1 of hVL22.101LC42 |
| 11 | protein | hVH22.101f |
| 12 | protein | hVH22.101HC9 |
| 13 | protein | hVL22.101LC17 |
| 14 | protein | hVL22.101LC21 |
| 15 | protein | hVL22.101LC27 |
| 16 | protein | hVL22.101LC41 |
| 17 | protein | hVL22.101LC42 |
| 18 | protein | SEQ ID NO 1 from WO2016092082-Example 1, histone 2A |
| 19 | protein | SEQ ID NO 2 from WO2016092082, histone 4 |
| 20 | protein | Shortened SEQ ID NO 2 from WO2016092082-Example 7, histone 4 |
| 21 | protein | Peptide no 4 (human histone 2A) (SEQ ID NO 24 from WO2011070172) |
| 22 | protein | Peptide no 6 (human histone 2A) (SEQ ID NO 26 from WO2011070172) |
| 23 | protein | Human heavy chain constant domain of IgG1 |
| 24 | protein | Human kappa chain constant domain |
| 25 | protein | msVH22.101 |
| 26 | protein | hVH22.101j |
| 27 | protein | hVH22.101HC7 |
| 28 | protein | hVH22.101HC8 |
| 29 | protein | hVH22.101HC10 |
| 30 | protein | msVL22.101 |
| 31 | protein | hVL22.101e |
| 32 | protein | hVL22.101g |
| 33 | protein | hVL22.101h |
| 34 | protein | hVL22.101i |
| 35 | protein | hVL22.101j |
| 36 | protein | CDR1 of msVL22.101 and hVL22.101g |
| 37 | protein | CDR1 of hVL22.101e |
| 38 | protein | CDR1 of hVL22.101h |
| 39 | protein | CDR1 of hVL22.101i |
| 40 | protein | CDR1 of hVL22. 101j |
| 41 | protein | CDR1 of hVL22.101LC16 |
| 42 | protein | CDR1 of hVL22.101LC19 |
| 43 | protein | CDR1 of hVL22.101LC20 |
| 44 | protein | CDR1 of hVL22.101LC22 |
| 45 | protein | CDR1 of hVL22.101LC23 |
| 46 | protein | CDR1 of hVL22.101LC24 |
| 47 | protein | CDR1 of hVL22.101LC25 |
| 48 | protein | CDR1 of hVL22.101LC26 |
| 49 | protein | CDR1 of hVL22.101LC37 |
| 50 | protein | CDR1 of hVL22.101LC38 |
| 51 | protein | CDR1 of hVL22.101LC39 |
| 52 | protein | CDR1 of hVL22.101LC40 |
| 53 | protein | msFibβ XG (SEQ ID NO 37 from WO2011070172) |
| 54 | protein | msVim XS/XI, (SEQ ID NO 38 from WO2011070172) |
| 55 | Protein | Region around CDR2 of msVL22.101 and hVL22.101(LC)y |
| 56 | Protein | Heavy chain constant domain of hCH22.101f |

### Brief Description of the Figures

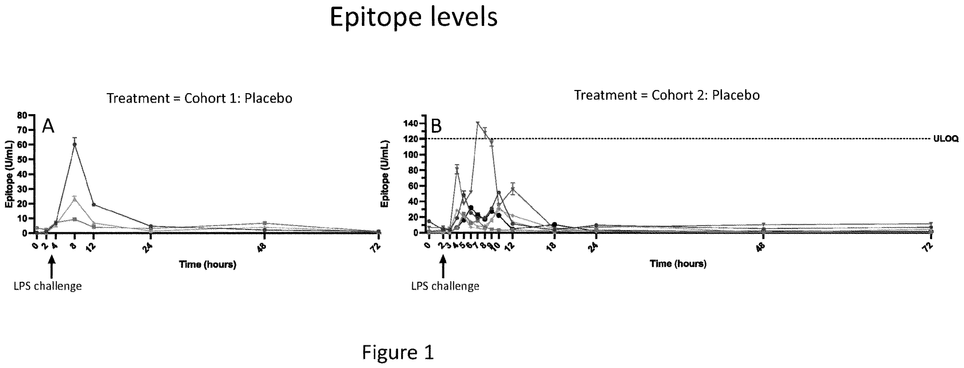

**Figure 1****.** CIT-013 epitope levels (Units/ml) in serum from LPS challenged healthy volunteers that have been treated with placebo. A) cohort 1(n=3) and B) cohort 2 (n=6). LPS was dosed at 2 ng/kg two hours after placebo treatment. The measurement was only performed in the placebo groups as the assay relied on an antibody binding the epitope for CIT-013 which would cross-compete with CIT-013 present in the serum of the volunteers dosed with CIT-013. LTLOQ = Upper limit of quantification.

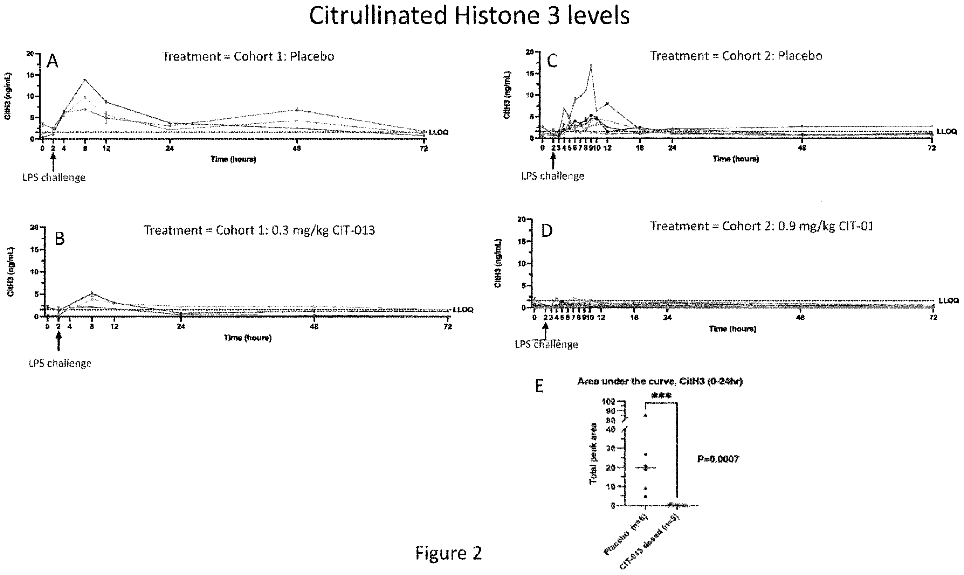

**Figure 2****.** Citrullinated histone 3 levels (ng/ml) in serum from two cohorts of LPS challenged healthy volunteers treated with either placebo or CIT-013. LPS was dosed at 2 ng/kg two hours after placebo or CIT-013 treatment. Cohort 1 has been treated with A) placebo (n=3) or B) 0.3 mg/kg CIT-013 (n=3). Cohort 2 has been treated with C) placebo (n=6) or D) 0.9 mg/kg CIT-013 (n=8). E) shows the calculated area under the curve (AUC) for cohort 2 placebo and CIT-013 treated healthy volunteers. A non-paired Mann-Whitney test was performed to calculate statistical differences. ***, p=0.00075. LLOQ is lower limit of quantification.

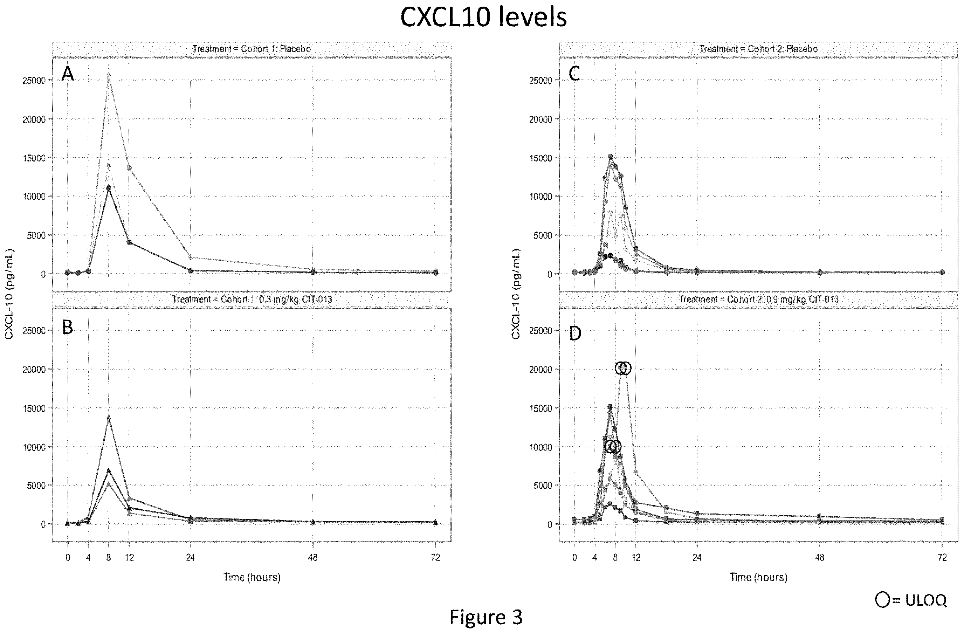

**Figure 3****.** CXCL10 levels (pg/ml) in plasma from two cohorts of LPS challenged healthy volunteers treated with either placebo or CIT-013. LPS was dosed at 2 ng/kg two hours after placebo or CIT-013 treatment. Cohort 1 has been treated with A) placebo (n=3) or B) 0.3 mg/kg CIT-013 (n=3). Cohort 2 has been treated with C) placebo (n=6) or D) 0.9 mg/kg CIT-013 (n=8). The circled datapoints at 20000 pg/ml indicate that the upper limit of quantification has been reached and that further dilution of the sample was not possible.

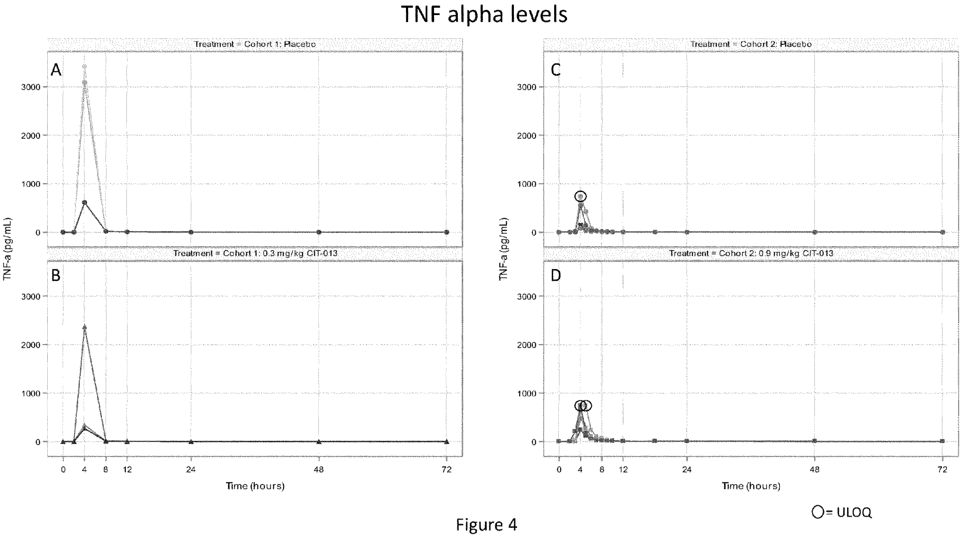

**Figure 4****.** TNF-alpha levels (pg/ml) in plasma from two cohorts of LPS challenged healthy volunteers treated with either placebo or CIT-013. LPS was dosed at 2 ng/kg two hours after placebo or CIT-013 treatment. Cohort 1 has been treated with A) placebo (n=3) or B) 0.3 mg/kg CIT-013 (n=3). Cohort 2 has been treated with C) placebo (n=6) or D) 0.9 mg/kg CIT-013 (n=8). Circled datapoints indicate that the upper limit of quantification had been reached and that further dilution of the sample was not possible.

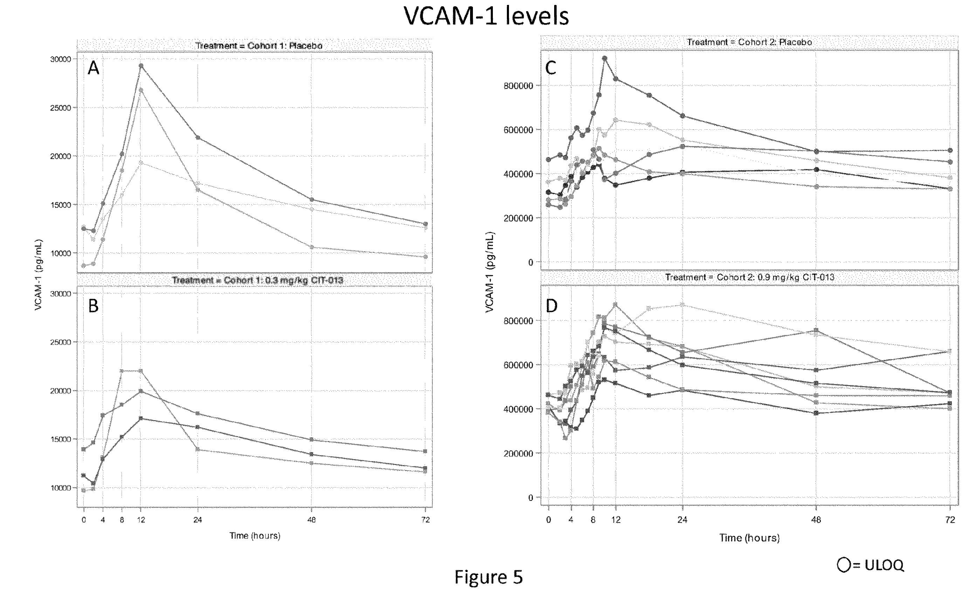

**Figure 5****.** VCAM-1 levels (pg/ml) in plasma from wo cohorts of LPS challenged healthy volunteers treated with either placebo or CIT-013. LPS was dosed at 2 ng/kg two hours after placebo or CIT-013 treatment. Cohort 1 has been treated with A) placebo (n=3) or B) 0.3 mg/kg CIT-013 (n=3). Cohort 2 has been treated with C) placebo (n=6) or D) 0.9 mg/kg CIT-013 (n=8).

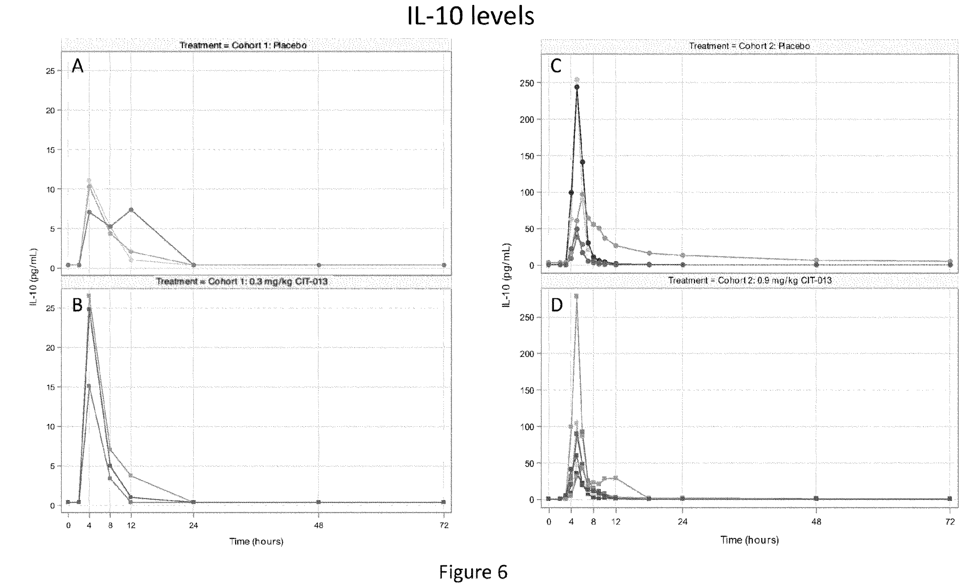

**Figure 6****.** IL-10 levels (pg/ml) in plasma from two cohorts of LPS challenged healthy volunteers treated with either placebo or CIT-013. LPS was dosed at 2 ng/kg two hours after placebo or CIT-013 treatment. Cohort 1 has been treated with A) placebo (n=3) or B) 0.3 mg/kg CIT-013 (n=3). Cohort 2 has been treated with C) placebo (n=6) or D) 0.9 mg/kg CIT-013 (n=8).

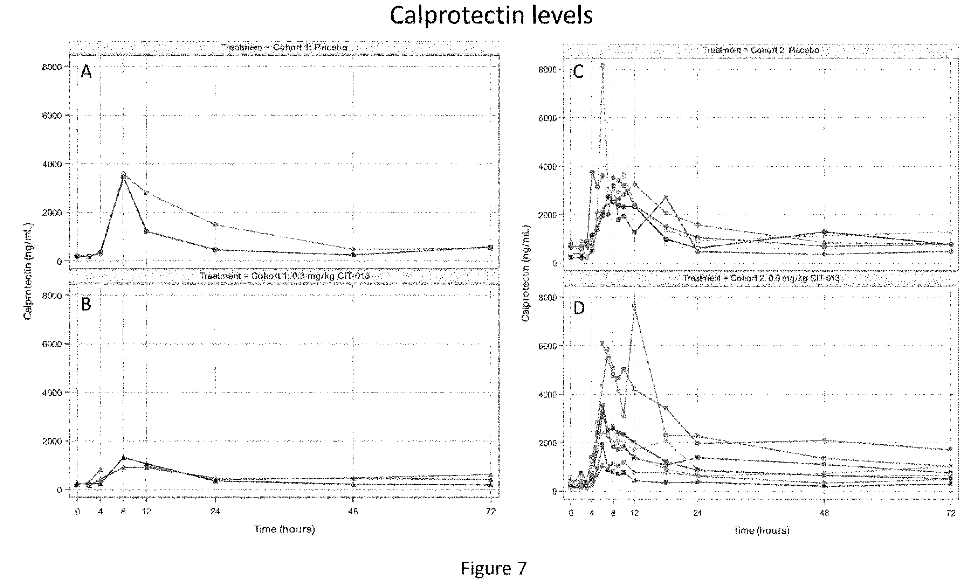

**Figure 7****.** Calprotectin levels (ng/ml) in plasma from two cohorts of LPS challenged healthy volunteers treated with either placebo or CIT-013. LPS was dosed at 2 ng/kg two hours after placebo or CIT-013 treatment. Cohort 1 has been treated with A) placebo (n=3) or B) 0.3 mg/kg CIT-013 (n=3). Cohort 2 has been treated with C) placebo (n=6) or D) 0.9 mg/kg CIT-013 (n=8).

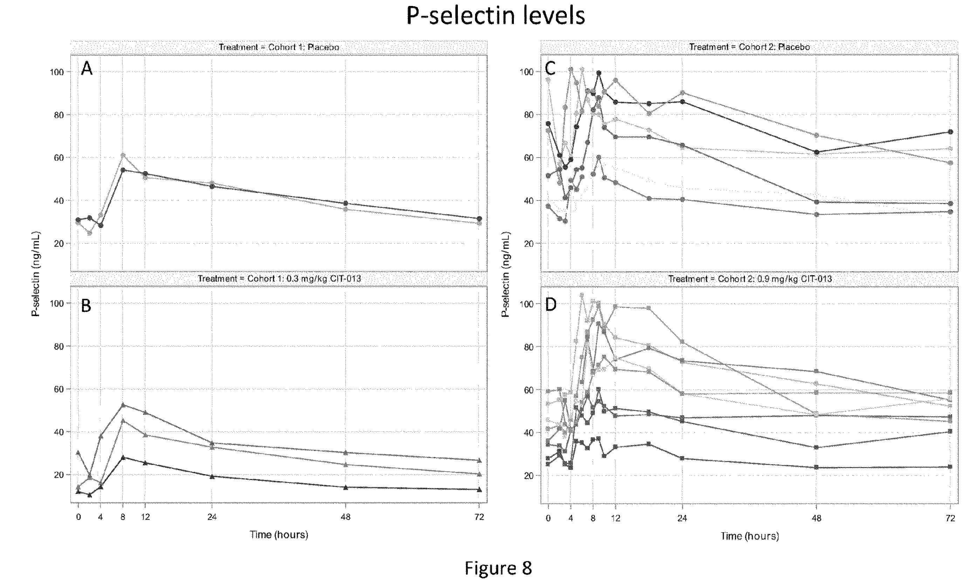

**Figure 8****.** P-selectin levels (ng/ml) in plasma from two cohorts of LPS challenged healthy volunteers treated with either placebo or CIT-013. LPS was dosed at 2 ng/kg two hours after placebo or CIT-013 treatment. Cohort 1 has been treated with A) placebo (n=2) or B) 0.3 mg/kg CIT-013 (n=3). Cohort 2 has been treated with C) placebo (n=6) or D) 0.9 mg/kg CIT-013 (n=8).

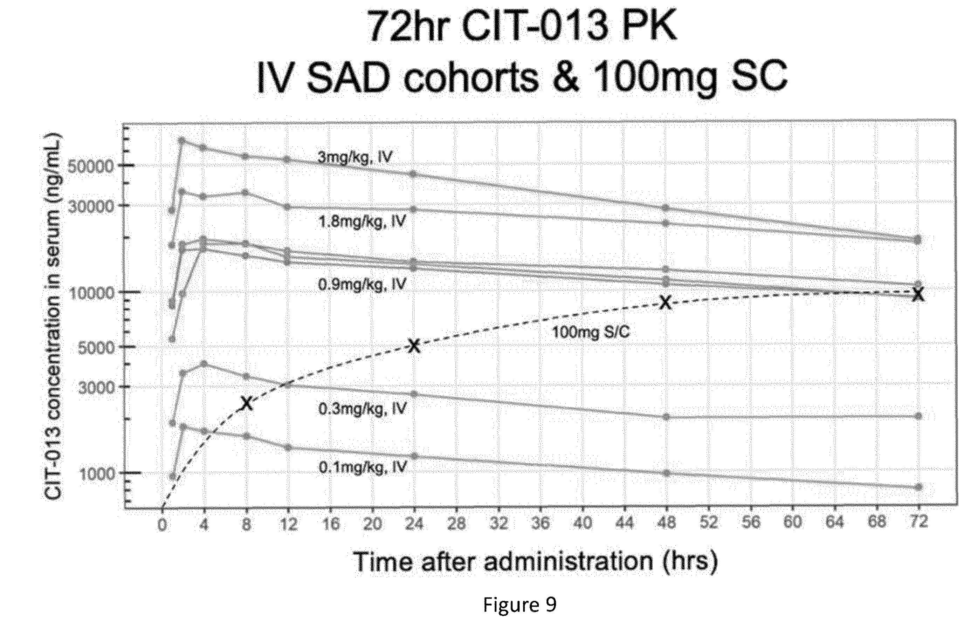

**Figure 9****:** Exposure of CIT-013 in the blood when administered intravenously (IV) or subcutaneously (SC). The graph shows the CIT-013 serum concentration over time when administered as a single IV dose of CIT-013 (5 different mg/kgs) or as a single dose of 100mg administered SC.

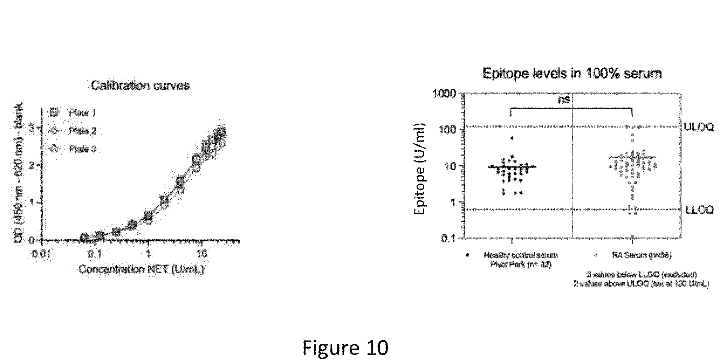

**Figure 10****:** Calibration curves from three different plates are depicted in Figure 10a. Figure 10b demonstrates the significant difference in citrullinated NET levels in serum samples from the Rheumatoid Arthritis (RA) cohort (results shown on the right) compared to serum from the Healthy Volunteer (HV) cohort (results shown on the left). No significance was determined using the Mann-Whitney test (unpaired; nonparametric; two tailed P-value and 95% confidence level). (P = 0.074)

### Detailed Description of the Invention

It is to be understood that different applications of the disclosed invention may be tailored to the specific needs in the art. It is also to be understood that the terminology used herein is for the purpose of describing particular embodiments of the invention only and is not intended to be limiting.

In addition, as used in this specification and the appended claims, the singular forms "a", "an", and "the" include plural references unless the content clearly dictates otherwise. Thus, for example, reference to "an antibody" includes "antibodies", and the like.

Where the term "comprising" is used herein, also provided is an embodiment "consisting essentially of" or "consisting of" what is set out.

The term "about" in relation to a point value indicated herein in one embodiment means that what is provided is within ± 10% of the stated point value. In one preferred embodiment, it is within ± 5%. In one more preferred embodiment, it is within ± 1%. When used in relation to a numerical range, in one embodiment the amount that each point value used to indicate the endpoint of a range me value is within any of those values.

All publications, patents and patent applications cited herein, whether supra or infra, are hereby incorporated by reference in their entirety.

### Doses and regimens

The present invention provides an antibody or binding fragment thereof that specifically binds to a citrullinated epitope on deiminated human histone 2A and/or histone 4 for use in a method of treating or preventing a disease associated with extracellular trap release from cells, such as an Neutrophil Extracellular Trap (NET)-associated pathology or an Eosinophil Extracellular Trap (EET) -associated pathology in a subject in need thereof, wherein the method comprises administering at least one dose that is equivalent to an intravenous administration of about 0.01 mg/kg to about 0.6 mg/kg of the antibody or binding fragment thereof to the subject

The present invention provides an antibody or binding fragment thereof that specifically binds to a citrullinated epitope on deiminated human histone 2A and/or histone 4 for use in a method of treating or preventing a disease associated with extracellular trap release from cells, such as an Neutrophil Extracellular Trap (NET)-associated pathology or an Eosinophil Extracellular Trap (EET) -associated pathology in a subject in need thereof, wherein the method comprises administering at least one dose of about 0.01 mg/kg to about 0.6 mg/kg of the antibody or binding fragment thereof to the subject. In one embodiment, at least one dose is from about 0.1 mg/kg to about 0.6 mg/kg. In another it is from about 0.1 mg/kg to about 0.5 mg/kg. In another, it is from about 0.1 mg/kg to about 0.4 mg/kg

In one preferred embodiment, at least one dose is about 0.02 mg/kg to about 0.5 mg/kg. In one more preferred embodiment, at least one dose is about 0.05 mg/kg to about 0.4 mg/kg. In one even more preferred embodiment, the at least one dose is about 0.1 mg/kg to about 0.3 mg/kg. An example of a preferred dose is 0.02 mg/kg. An example of a preferred at least one dose is about 0.05 mg/kg. An example of a preferred at least one dose is about 0.1 mg/kg. A further example of a preferred at least one dose is about 0.2 mg. A further example of a preferred at least one dose is about 0.3 mg/kg. In a preferred embodiment the at least one dose is administered by injection or infusion, more preferably via injection. In a particularly preferred embodiment, it is administered subcutaneously or intravenously. In a preferred embodiment it is administered subcutaneously. In a further preferred embodiment, it is administered intravenously.

In one embodiment, from 1 to 12 doses will be administered to the subject. In one embodiment, from 1 to 10 doses will be administered. In one embodiment, from 2 to 8 doses will be administered. In another embodiment, from 3 to 7 doses will be administered. In one embodiment, 1, 2, 3, 4, 5, 6, 7 or 8 doses will be administered. In a more preferred embodiment, 5, 6, or 7 doses will be administered. In a particularly preferred embodiment, 6 doses will be administered. In an alternative embodiment, 1, 2, or 3 doses will be administered. In one embodiment, 1 or 2 doses will be administered. In one embodiment, a single dose of antibody will be administered. In an alternative embodiment, the antibody is dosed chronically as a maintenance therapy. In an alternative embodiment, the dosing of the antibody will continue as a maintenance therapy. In an alternative embodiment, the dosing of the antibody will continue as long as considered needed. In another embodiment, the dosing of the antibody will continue until the condition of the subject has improved.

In embodiments more than one dose of the antibody or binding fragment is administered, wherein the interval between doses is from 1 week to 6 months. In embodiments more than one dose of the antibody or binding fragment is administered, wherein the interval between doses is from 1 week to 5 months. In embodiments more than one dose of the antibody or binding fragment is administered, wherein the interval between doses is from 1 week to 4 months. In embodiments more than one dose of the antibody or binding fragment is administered, wherein the interval between doses is from 1 week to 3 months. In one embodiment, the interval between doses is from 1 week to six weeks. In one embodiment, doses are administered at about 1 to 4 week intervals. In one embodiment, doses are administered at about monthly intervals. In a preferred embodiment, doses are administered from 1 to 3 weeks apart. In a particularly preferred embodiment, doses will be administered at about 2 weekly intervals. In one embodiment, doses are administered at about weekly, fortnightly, or monthly intervals.

In one preferred embodiment, the present invention provides an antibody or binding fragment thereof that specifically binds to a citrullinated epitope on deiminated human histone 2A and/or histone 4 for use in a method of treating or preventing a disease associated with extracellular trap release from cells, such as a Neutrophil Extracellular Trap (NET)-associated pathology or an Eosinophil Extracellular Trap (EET) -associated pathology in a subject in need thereof, wherein the method comprises administering from 1 to 10 doses of the antibody or binding fragment thereof to the subject, wherein each dose is about 0.02 mg/kg to about 0.5 mg/kg, wherein the antibody is administered by injection or infusion. Preferably the antibody or binding fragment is administered subcutaneously or intravenously. Particularly preferably it is administered subcutaneously. In one preferred embodiment wherein at least two doses are administered, the interval between doses is from 1 week to 3 months. In a particularly preferred embodiment, the interval is from 1 week to 3 weeks. In a further particularly preferred embodiment, from 4 to 8 doses are administered, preferably at 1 week to 3 week intervals.

In one preferred embodiment, the present invention provides an antibody or binding fragment thereof that specifically binds to a citrullinated epitope on deiminated human histone 2A and/or histone 4 for use in a method of treating or preventing a disease associated with extracellular trap release from cells, such as a Neutrophil Extracellular Trap (NET)-associated pathology or an Eosinophil Extracellular Trap (EET) -associated pathology in a subject in need thereof, wherein the method comprises administering from 1 to 10 doses of the antibody or binding fragment thereof to the subject, wherein each dose is about 0.05 mg/kg to about 0.5 mg/kg, wherein the antibody is administered by injection or infusion. Preferably the antibody or binding fragment is administered subcutaneously or intravenously. Particularly preferably it is administered subcutaneously. In one preferred embodiment wherein at least two doses are administered, the interval between doses is from 1 week to 3 months. In a particularly preferred embodiment, the interval is from 1 week to 3 weeks. In a further particularly preferred embodiment, from 4 to 8 doses are administered, preferably at 1 week to 3 week intervals.

In one preferred embodiment, the present invention provides an antibody or binding fragment thereof that specifically binds to a citrullinated epitope on deiminated human histone 2A and/or histone 4 for use in a method of treating or preventing a disease associated with extracellular trap release from cells, such as a Neutrophil Extracellular Trap (NET)-associated pathology or an Eosinophil Extracellular Trap (EET) -associated pathology in a subject in need thereof, wherein the method comprises administering from 1 to 10 doses of the antibody or binding fragment thereof to the subject, wherein each dose is about 0.1 mg/kg to about 0.4 mg/kg, wherein the antibody is administered by injection or infusion. Preferably the antibody or binding fragment is administered subcutaneously or intravenously. Particularly preferably it is administered subcutaneously. In one preferred embodiment wherein at least two doses are administered, the interval between doses is from 1 week to 3 months. In a particularly preferred embodiment, the interval is from 1 week to 3 weeks. In a further particularly preferred embodiment, from 4 to 8 doses are administered, preferably at 1 week to 3 week intervals.

In one preferred embodiment, the present invention provides an antibody or binding fragment thereof that specifically binds to a citrullinated epitope on deiminated human histone 2A and/or histone 4 for use in a method of treating or preventing a disease associated with extracellular trap release from cells, such as a Neutrophil Extracellular Trap (NET)-associated pathology or an Eosinophil Extracellular Trap (EET) -associated pathology in a subject in need thereof, wherein the method comprises administering from 4 to 8 doses of the antibody or binding fragment thereof to the subject, wherein each dose is about 0.02 mg/kg to about 0.3 mg/kg, wherein the antibody is administered by injection or infusion, wherein the interval between doses is from 1 week to 1 month. In a particularly preferred embodiment, the administration is at 1 week to 3 week intervals.

In one embodiment, a fixed dose is administered. In one embodiment, preferred fixed doses are administered subcutaneously with a fixed dose of 2 mg to 100 mg. In one embodiment, preferred fixed doses are administered subcutaneously with a fixed dose of 2 mg to 90 mg. In one embodiment, preferred fixed doses are administered subcutaneously with a fixed dose of 2 mg to 80 mg. In one embodiment, preferred fixed doses are administered subcutaneously with a fixed dose of 2 mg to 70 mg. In one embodiment, preferred fixed doses are administered subcutaneously with a fixed dose of 2 mg to 60 mg. In one embodiment, preferred fixed doses are administered subcutaneously with a fixed dose of 2 mg to 50 mg. In one preferred embodiment, the fixed dose is administered subcutaneously at a fixed dose of from 2 to 40 mg. In one preferred embodiment, the fixed dose is administered subcutaneously at a fixed dose of from 2 to 30 mg. In one preferred embodiment, the fixed dose is administered subcutaneously at a fixed dose of from 2 to 20 mg. In one preferred embodiment, the fixed dose is administered subcutaneously at a fixed dose of from 8 to 30 mg. In one preferred embodiment, the fixed dose is administered subcutaneously at a fixed dose of from 8 to 20 mg. In one embodiment at least two such fixed doses may be administered at any of the intervals set out herein. In one embodiment, from 2 to 10 fixed doses may be administered at internals of 1 to 6 weeks apart. In one preferred embodiment, from 4 to 8 fixed doses may be administered. In one preferred embodiment, the 4 to 8 fix doses are administered at an interval of from 1 week to 1 month apart.

As shown in Figure 9, subcutaneous administration of 100mg CIT-013 reaches an exposure level in the serum at 72 hours post administration of about 10,000 ng/ml. This exposure level is equivalent to that reached at 72 hrs after an intravenous (IV) administration of 0.7 mg/kg of CIT-013. The IV curves indicate that the ng/ml exposures are more or less linear. Therefore, if twice as much CIT-013 IV is administered twice as much is detected in the serum at 72 hrs. It is expected therefore that a 20 mg subcutaneous administration of CIT-013 would result in an exposure level in the serum at 72 hours post administration of about 2,000 ng/ml, which equates to the exposure level seen at 72 hrs after an IV administration of 0.3 mg/kg of CIT-013. It is expected therefore that an 8 mg subcutaneous administration of CIT-013 would result in an exposure level in the serum at 72 hours post administration of about 800 ng/ml, which equates to the exposure level seen at 72 hrs after an IV administration of 0.1 mg/kg of CIT-013.

### Cytokines and markers

In a preferred embodiment, the amount of antibody administered results in either an increase or decrease in the amount of at least one cytokine or other marker. One of the advantages of the present invention is that it may result in a particular pattern of cytokine release or other markers.

In one preferred embodiment, the invention increases the level of IL-10 in the subject. That is the level of IL-10 after administration of the antibody is greater than the base level of IL-10 in the subject before any antibody has been administered. Hence, in one embodiment, administration of the antibody or binding fragment results in stimulation of IL-10. The IL-10 level measure may be that in plasma.

In one preferred embodiment, the invention results in suppression of the release of particular cytokines or markers. That is the level of cytokine or marker release is less than the baseline level prior to administration of the antibody. In one embodiment, the administration of the antibody or binding fragment suppresses the level of at least one of calprotectin, P-selectin, CXCL10, TNF-alpha, and VCAM in the subject. In one embodiment, the level of at least two of the cytokines or markers is suppressed. In one embodiment, the level of at least three of the cytokines or markers is suppressed. In one embodiment, the level of at least four of the cytokines or markers is suppressed. Preferably, the level of all five is suppressed.

In one preferred embodiment, the administration of the antibody results in any increase in the level of IL-10, but suppresses the level of at least one of calprotectin, P-selectin, CXCL10, TNF-alpha, and VCAM in the subject. Preferably, at least two of calprotectin, P-selectin, CXCL10, TNF-alpha, and VCAM are suppressed. More preferably, the level of at least three of calprotectin, P-selectin, CXCL10, TNF-alpha, and VCAM is suppressed. More preferably, the level of at least four of calprotectin, P-selectin, CXCL10, TNF-alpha, and VCAM is suppressed. More preferably, the level of all five of calprotectin, P-selectin, CXCL10, TNF-alpha, and VCAM is suppressed.

Levels of IL-10, calprotectin, P-selectin, CXCL10, TNF-alpha, and VCAM may be measured by any suitable means, including via commercially available kits. In a preferred embodiment, the methods used are those in the Examples of the present application.

### Disorders

The present invention relates to antibodies or binding fragments thereof that specifically bind to a citrullinated epitope on deiminated human histone 2A and/or histone 4 for use in the treatment or prevention of diseases associated with extracellular trap release from cells, such as Neutrophil Extracellular Trap (NET)-associated pathologies or Eosinophil Extracellular Trap (EET) -associated pathologies.

The antibodies or binding fragments thereof of the present invention, or the pharmaceutical compositions as defined herein, are particularly suited for use in the treatment or prevention of pathologies associated with citrullination, such as NET-associated pathologies and inflammatory conditions and/or EET-associated pathologies.

The present invention also encompasses a method of treating a patient comprising administering a therapeutically effective amount of an antibody or binding fragment thereof as defined herein or the pharmaceutical composition as defined herein to a patient, optionally to treat or prevent pathologies associated with citrullination, such as NET-associated pathologies and inflammatory conditions and/or EET-associated pathologies.

The present invention also encompasses an antibody or binding fragment thereof as defined herein or the pharmaceutical composition as defined herein for use in the manufacture of a medicament for the prevention or treatment of pathologies associated with citrullination, such as NET-associated pathologies and inflammatory conditions and/or EET-associated pathologies.

The present invention also encompasses a pharmaceutical composition comprising the antibody or binding fragment thereof of the present invention for treating or preventing pathologies associated with citrullination, such as NET-associated pathologies and inflammatory conditions and/or EET-associated pathologies.

A pathology associated with citrullination can be defined as any disease or condition where citrullination is associated with the pathological state of the disease or condition. Whether or not citrullination plays a role in the pathogenesis of the disease, may be easily determined by a skilled person using routine tests available in the art. For example, these diseases may be characterized by the presence of an abnormal level of citrullinated proteins in affected or disease-related tissue. Such may be accomplished by an immunological test such as a Western blot or an ELISA wherein the affected tissue is used as an antigen and citrullination of that antigen may be detected with the aid of an anti-citrulline antibody as described herein. Alternatively, a person skilled in the art can use Proteomic applications such as mass spectrometry analysis to compare the level and type of citrullination in a diseased versus healthy tissue from affected patients.

NET-associated pathologies can be considered as pathologies associated with citrullination. NET-associated pathologies can be defined as a disease or condition where the formation of NETs and NETosis is associated with the pathological state of the disease or condition. Whether or not NET formation and NETosis plays a role in the pathogenesis of the disease may be easily determined by a skilled person using routine tests available in the art. For example, these diseases may be characterized by the presence of NETs in relevant tissues.

The invention therefore relates to antibodies or binding fragments thereof for use in the treatment or prevention of NET-associated pathologies.

The invention therefore relates to a method of treating a patient in need thereof with a therapeutically effective amount of the antibody or binding fragments thereof of the present invention, wherein the patient is suffering from a NET-associated pathology.

Examples of NET-associated pathologies include inflammatory conditions or diseases, ocular inflammatory diseases, cardiovascular diseases, respiratory diseases, wound healing, skin diseases, autoimmune diseases, cancer, and organ-health after transplant.

"Inflammatory Conditions" or Inflammatory diseases" refers to any of a number of conditions or diseases, which are characterized by vascular changes: edema and infiltration of neutrophils (e.g., acute inflammatory reactions); infiltration of tissues by mononuclear cells; tissue destruction by inflammatory cells, connective tissue cells and their cellular products; and attempts at repair by connective tissue replacement (e.g., chronic inflammatory reactions). Such diseases are for instance inflammatory arthritis, including rheumatoid arthritis and osteoarthritis, SLE, lupus, sepsis, vasculitis, multiple sclerosis, psoriatic arthritis, psoriasis, hidradenitis supperativa, Alzheimer's disease, autoimmune hepatitis, juvenile idiopathic arthritis, myositis (polymyositis and dermatomyositis), Sjögren's disease, spondyloarthropathy, multiple system atrophy, heart failure, atherosclerosis, Parkinson's disease, Lewy body dementia, idiopathic pulmonary fibrosis, dry eye disease, uveitis, nongranulomatous uveitis, granulomatous uveitis, dermatitis, atopic dermatitis, and lung diseases such as asthma, acute respiratory distress syndrome (ARDS) including but not limited to coronovirus induced ARDS, COPD and bronchitis, thrombotic diseases and cardiovascular diseases. Nongranulomatous uveitis can be associated with neutrophil dominant inflammation, granulomatous uveitis can be associated with macrophage dominant inflammation.

NETs play a role in autoimmune diseases pathology, including RA, SLE and vasculitis. The pathway by which the therapeutic antibody or binding fragment thereof improves the disease is likely via the inhibition of NETosis, accelerating the clearance of remnants of NETs and NET-ing neutrophils, including toxic histones, and other auto-antigens from tissue and circulation, the clearance toxic histones from tissue and circulation. For many of several autoimmune diseases it has been shown that the pathology improves in PAD knock-out models or in wild-type animals treated with a PAD inhibitor or DNase, meaning that there is a strong correlation with the number of NETs in tissue and circulation and disease severity.
Thus, inflammatory conditions or diseases and autoimmune diseases can be treated by the antibodies and binding fragments thereof described in the present invention.

In a preferred embodiment, the diseases to be treated are NET-associated pathologies such as SLE, lupus, sepsis, vasculitis, inflammatory arthritis, rheumatoid arthritis and osteoarthritis, psoriasis, hidradenitis suppurativa Alzheimer's disease, autoimmune hepatitis, juvenile idiopathic arthritis, myositis (polymyositis and dermatomyositis), Sjögren's disease, Anti-phospholipid Syndrome, Bechet's disease, spondylitis, spondyloarthropathy, multiple system atrophy, Parkinson's disease, Lewy body dementia asthma, allergic rhinovirus exacerbated asthma, allergic asthma, cystic fibrosis, fibrosis and idiopathic pulmonary fibrosis, heart failure, atherosclerosis, dry eye disease, uveitis, nongranulomatous uveitis, granulomatous uveitis, dermatitis, atopic dermatitis, COPD, bronchitis, or other NET-associated pathologies such as wound healing in diabetes, cancer, cancer metastasis, and transplant organ health *in vivo* or *ex vivo.*

In a preferred embodiment, the diseases to be treated are inflammatory conditions such as SLE, lupus, sepsis, vasculitis, inflammatory arthritis, rheumatoid arthritis and osteoarthritis, psoriasis, hidradenitis suppurativa Alzheimer's disease, autoimmune hepatitis, juvenile idiopathic arthritis, myositis (polymyositis and dermatomyositis), Sjögren's disease, Anti-phospholipid Syndrome, Bechet's disease, spondylitis, spondyloarthropathy, multiple system atrophy, Parkinson's disease, Lewy body dementia asthma, allergic rhinovirus exacerbated asthma, allergic asthma, acute respiratory distress syndrome, cystic fibrosis, fibrosis, idiopathic pulmonary fibrosis, heart failure, atherosclerosis, dry eye disease, uveitis, nongranulomatous uveitis, granulomatous uveitis, dermatitis, atopic dermatitis, COPD, bronchitis.

In one preferred embodiment, the NET-associated disorder is a tauopathy. In a particularly preferred embodiment, the disorder is Parkinson's Disease.

The methods disclosed herein may be for the diagnosis, treatment or prevention of any disease or condition which includes an EET-associated pathology. An EET-associated pathology typically means a pathology which is mediated in whole or in part by the formation of EETs. Such a pathology is typically present in any disease or condition which is mediated in whole or in part, or preferably which is mediated primarily, by eosinophils. Such diseases or conditions may be described herein as eosinophilic or eosinophil-associated. Therefore, put another way, the methods disclosed herein may be for the diagnosis, treatment or prevention of an eosinophilic disease or condition.

An eosinophilic disease or condition may be defined as a disease or condition in which eosinophils are present in elevated numbers in the tissue or organ affected, relative to the same tissue or organ in a healthy individual. Eosinophilic diseases and conditions may include: an eosinophilic disease or condition of the skin; a respiratory eosinophilic disease or condition; a gastro-intestinal eosinophilic disease or condition; an allergic disease or condition; or a helminth, fungal, viral, or bacterial infection.

Eosinphilic diseases or conditions of the skin include Bullous Pemphigoid (PB), Atopic dermatitis (AD) and Chronic spontaneous Urticaria (CSU), allergic contact dermatitis, and eosinophilic cellulitis (also called Well's syndrome).

Respiratory eosinophilic diseases or conditions include Eosinophilic Asthma, Nasal Polyps, Chronic RhinoSinusitis with Nasal Polyposis (CRSwNP), Allergic sinusitis, Allergic rhinisitis, Allergic bronchopulmonary aspergillosis (a fungal infection), Eosinophilic chronic rhinosinusitis, Tropical pulmonary eosinophilia (typically a respiratory Helminth infection).

Gastro-intestinal eosinophilic diseases or conditions include Eosinophilic Esophagitis (EoE), Eosinophilic gastritis (stomach - EG), Eosinophilic gastroenteritis (stomach and small intestine - EGE), Eosinophilic enteritis (small intestine), Eosinophilic colitis (large intestine - EC), and a gastro-intestinal helminth infection such as Ascariasis or Trichinosis.

Other eosinophilic diseases or conditions include HyperEosinophilic Syndrome (HES - affects blood and various organs), Eosinophilic Granulomatosis with PolyAngitis (EGPA - affects various organs including blood vessels) and Eosinophilic otitis media (EOM - affects the middle ear), and Drug Reaction with Eosinophilic & Systemic Symptoms (DRESS - affects various organs).

In one preferred embodiment, the disease to be treated or prevented is arteriosclerosis. In another embodiment vasculitis is treated.

The methods disclosed herein may be for the diagnosis, treatment or prevention of any of the above-listed eosinophilic diseases or conditions. Particularly preferred eosinophilic diseases or conditions include those in which the presence of EETs has been directly confirmed. Such disease and conditions include but are not limited to: Bullous Pemphigoid, Atopic dermatitis, allergic contact dermatitis, Eosinophilic Asthma, Chronic RhinoSinusitis with Nasal Polyposis (CRSwNP), Allergic sinusitis, Allergic bronchopulmonary aspergillosis, Eosinophilic chronic rhinosinusitis, Eosinophilic Esophagitis (EoE), HyperEosinophilic Syndrome (HES), Eosinophilic Granulomatosis with PolyAngitis (EGPA), Eosinophilic otitis media (EOM), and Drug Reaction with Eosinophilic & Systemic Symptoms (DRESS).

The most preferred eosinophilic diseases or conditions are those in which a correlation between EETs and disease incidence and/or severity has been directly observed. Such disease and conditions include but are not limited to: Eosinophilic Asthma, Chronic RhinoSinusitis with Nasal Polyposis (CRSwNP), Eosinophilic chronic rhinosinusitis, and Eosinophilic otitis media (EOM).

The presence of EETs and/or a role for eosinophils in diseases such as those discussed above is well-established in the art. See for example: Williams, T. L et al. (2020). "NETs and EETs, a Whole Web of Mess". Microorganisms, 8(12), 1925 and Mukherjee, M., et al. (2018). Eosinophil Extracellular Traps and Inflammatory Pathologies-Untangling the Web!. Frontiers in immunology, 9, 2763. The invention is suitable for the treatment, prevention or diagnosis of any disease recited in these documents, which are incorporated by reference.

The methods disclosed herein may also be for the diagnosis, treatment or prevention of an EET-associated pathology in a disease or condition which is only partly mediated by eosinophils. For example, diseases such as Chronic Obstructive Pulmonary Disease (COPD), Crohn's disease, ulcerative colitis, dermatitis herpetiformis, thrombosis, and atherosclerosis may exhibit multiple pathologies caused by multiple cell types, and so may not be defined as "eosinophilic". However, they may nonetheless exhibit EET-associated pathology and thus be diagnosed, treated or prevented by the methods disclosed herein.

In one preferred embodiment, the present invention is employed to treat a lung disorder. In particular, the present invention provides a method of treating or preventing a lung disorder comprising administering an antibody or binding fragment thereof that specifically binds to a citrullinated epitope on deiminated human histone 2A and/or histone 4 to a subject suffering, or at risk of, said lung disorder. In one embodiment, the antibody or binding fragment is any of those described herein. In a preferred embodiment, the lung disorder may be an inflammatory lung disorder. In one embodiment, the lung disorder is one characterized by an influx of inflammatory cells to the lung compared to a healthy subject without the disorder. For example, the condition may be in one embodiment characterized by an influx of white blood cells to the lung. In one embodiment, the lung disorder is characterized by an influx of granulocytes to the lung, in particular eosinophils and/or neutrophils to the lung.

In one embodiment, the lung condition is characterized by the subject showing poor symptom responsiveness to corticosteroids. In particular, in one embodiment, the approach provided is used to treat a subject with a lung condition showing poor responsiveness to dexamethasone. In another embodiment, the subject is treated both with an antibody or binding fragment thereof that specifically binds to a citrullinated epitope on deiminated human histone 2A and/or histone 4 and also a corticosteroid. In one embodiment, a subject is treated with both the antibody (or binding fragment) and dexamethasone. In one embodiment, combining the two may help augment the effect of the corticosteroid.

In one embodiment, the method of treating or preventing the lung disorder results in a reduction in the presence of NETs. In another embodiment, the method results in a reduction in the presence of EETs. In a preferred embodiment, the method may result in a reduction of both NETs and EETs in the lungs of the subject. In one embodiment, the method results in a reduction of the formation of NETs and/or EETs.

The methods may be used to treat any suitable lung disorder, particularly an inflammatory lung disorder. In one embodiment, the lung disorder is selected from COPD, bronchitis, emphysema, cystic fibrosis, fibrosis and idiopathic pulmonary fibrosis, and asthma. In a preferred embodiment, the condition is asthma. It may be that the subject has severe asthma. In one particularly preferred embodiment, the lung disorder may be allergic asthma. In one preferred embodiment, the lung disorder is allergic asthma involving house dust mite allergy. In one embodiment, the lung disorder is asthma characterised by the presence of a raised number of eosinophils and/or neutrophils. In one embodiment a method of the present invention may be used to treat a lung condition with increased numbers of infiltrating eosinophils. In another embodiment, a method of the present invention may be used to treat a lung condition with increased numbers of infiltrating neutrophils. In another embodiment, the subject has increased numbers of infiltrating eosinophils and neutrophils. In one embodiment the subject may have neutrophilic asthma. In another embodiment, the subject may have eosinophilic asthma. In one embodiment, the subject may have type 2 asthma. In another embodiment, the subject may have non-type 2 asthma.

In one embodiment, Bronchoalveolar lavage (BAL) may be used as a way to assess the presence of inflammatory cells in the lung. In one embodiment BAL may be used as a way to measure total white blood cell counts in the bronchoalveolar space. In one embodiment, BAL may be used as a way to measure the number of neutrophils and/or eosinophils in the bronchoalveolar space. In one embodiment, a method of the present invention will result in a reduced neutrophil count in BAL from the subject compared to the count prior to treatment. In another embodiment, the treatment will result in a reduced eosinophil count in BAL from the subject compared to the count prior to treatment or over the course of the treatment. In one embodiment, both eosinophil and neutrophil counts will be reduced. In one embodiment, the total granulocyte count in BAL will be reduced as a result of treatment. In one embodiment the invention may result in a reduction of perivascular infiltrating neutrophils, perivascular mononuclear cells and/or bronchiolar infiltrating neutrophils. Treatment with an antibody or binding fragment thereof as described herein may also result in a reduction in citrullinated histone, for instance as measured in BAL, particularly citrullinated histone 3 in BAL.

Typically, antibodies or compositions comprising them are administered to a subject already suffering from a disorder or condition, in an amount sufficient to cure, alleviate or partially arrest the condition or one or more of its symptoms. Such therapeutic treatment may result in a decrease in severity of disease symptoms, or an increase in frequency or duration of symptom-free periods. An amount adequate to accomplish this is defined as "therapeutically effective amount". Effective amounts for a given purpose will depend on the severity of the disease or injury as well as the weight and general state of the subject. As used herein, the term "subject" includes any human.

### Antibodies

The present invention relates to antibodies or binding fragments thereof that specifically bind to a citrullinated epitope on deiminated human histone 2A and/or histone 4 and in particular to dosages of such antibodies or binding fragments thereof to be employed in treating or preventing a Neutrophil Extracellular Trap associated pathology (a NET-associated pathology). The present section provides examples of possible antibodies or binding fragments thereof that are provided for use as set out herein and also to be employed in the methods set out herein. For the sake of brevity though, the present section will simply refer to antibodies, but both the antibodies for use as set out herein and methods comprising their administration are provided, as is use of the antibodies in the manufacture of medicaments.

Citrulline is an amino acid that is not incorporated into proteins during normal translation, however, it may be generated by post-translational modification of an arginine residue by enzymes such as Peptidylarginine deiminases (PAD); (EC 3.5.3.15). In mammals (humans, mice and rats), five PAD isotypes (PAD1 - PAD6; 'PAD4' and 'PAD5' are used for the same isotype), each encoded by a distinct gene, have been identified thus far.

Citrullination of histone 2A and/or histone 4 is associated with the formation of NETs. The downstream pathological effects of NET formation can be numerous. For example, there can be autoantigen exposure to the extracellular space and the subsequent production of pathological autoantibodies by the subject. NET-derived histones can be toxic to the vascular wall and organs leading to vascular damage and organ failure. NETs can lead to the formation of autoantigen/autoantibody immune complexes, which enhance further inflammation, in for example the kidney of SLE patients. NETs are also involved in metastasis in cancer progression.

Any suitable antibody or binding fragment thereof that specifically binds to a citrullinated epitope on deiminated human histone 2A and/or histone 4 may be employed in the present invention. Deimination of human histone 2A and 4 can be carried out by enzymes such as PADs, for example PAD2 and PAD4. In a specific embodiment, the antibodies or binding fragments thereof according to the invention specifically bind to a citrullinated epitope on deiminated human histone 2A and/or histone 4, wherein the epitope comprises a peptide selected from the group consisting of SEQ ID NOs: 18, 19, 20, 21 and 22. The antibodies or binding fragments thereof may also bind to epitopes comprising the peptides of SEQ ID NO: 53 or 54.

The term "antibodies", "antibody" or " binding fragment thereof" as used herein refers to a structure, preferably a protein or polypeptide structure, capable of specific binding to a target molecule often referred to as "antigen". The antibody molecule as employed herein refers to an antibody or binding fragment thereof. In a particularly preferred embodiment, the term 'antibody' as used herein generally relates to intact (whole) antibodies i.e. comprising the elements of two heavy chains and two light chains. The antibody may comprise further additional binding domains for example as per the molecule DVD-Ig as disclosed in WO 2007/024715, or the so-called (FabFv)₂Fc described in WO2011/030107. Thus 'antibody' as employed herein includes mono-, bi-, tri- or tetra-valent full-length antibodies. Reference to an "antibody" herein specifically encompasses an antigen binding fragment being employed, unless it is clear from the context used that is not the case.

An antibody or binding fragment thereof may be selected from the group consisting of single chain antibodies, single chain variable fragments (scFvs), variable fragments (Fvs), fragment antigen-binding regions (Fabs), recombinant antibodies, monoclonal antibodies, fusion proteins comprising the antigen-binding domain of a native antibody or an aptamer, single-domain antibodies (sdAbs), also known as VHH antibodies, nanobodies (Camelid-derived single-domain antibodies), shark IgNAR-derived single-domain antibody fragments called VNAR, diabodies, triabodies, Anticalins, aptamers (DNA or RNA) and active components or fragments thereof.

Multi-valent antibodies may comprise multiple specificities e.g. bispecific or may be monospecific.

In one embodiment, rather than a full length antibody an antigen-binding fragment thereof may be employed. Binding fragments of antibodies include single chain antibodies (i.e. a full-length heavy chain and light chain); Fab, modified Fab, Fab', modified Fab', F(ab')2, Fv, Fab-Fv, Fab-dsFv, single domain antibodies (e.g. VH or VL or VHH), scFv, mono-, bi-, tri- or tetra-valent antibodies, Bis-scFv, diabodies, tribodies, triabodies, tetrabodies and epitope-binding fragments of any of the above (see for example Holliger P and Hudson PJ, 2005, Nat. Biotechnol., 23,: 1126-1136; Adair JR and Lawson ADG, 2005, Drug Design Reviews - Online, 2, 209-217). The methods for creating and manufacturing these antibody fragments are well known in the art (see for example Verma R et al., 1998, J. Immunol. Methods, 216, 165-181). The Fab-Fv format was first disclosed in WO2009/040562 and the disulphide-stabilised versions thereof, the Fab-dsFv was first disclosed in WO2010/035012. Other antibody fragments for use in the present invention include Fab and Fab' fragments.

In one embodiment, the antibody is selected from a full-size antibody, Fab, F(ab')2, single-chain Fv fragment, single-domain VHH, Single domain VH or single domain VL.

IgG1 (e.g. IgG1/kappa) antibodies having an IgG1 heavy chain and a light chain may advantageously be used in the invention. However, other human antibody isotypes are also encompassed by the invention, including IgG2, IgG3, IgG4, IgM, IgA1, IgA2, IgAsec, IgD and IgE in combination with a kappa or lambda light chain. Also, all animal-derived antibodies of various isotypes can be used in the invention.

The term: "specifically binds to citrulline" or "specifically binds to a citrullinated epitope" in this context means that the antibody or binding fragment thereof binds to a structure such as a peptide containing a citrulline residue whereas the antibody or binding fragment thereof binds less strongly or preferably not at all with the same structure containing an arginine residue instead of the citrulline residue. The term peptide should be interpreted as a structure that is capable of presenting the citrulline residue in the correct context for immunoreactivity with the antibodies or binding fragments thereof as described herein, preferably in the same context as it appears in the human or animal body, preferably in the context of a native polypeptide.

The antibodies or binding fragments thereof of the invention specifically bind to a citrullinated epitope on deiminated human histone 2A and/or histone 4. The binding of antibodies or binding fragments thereof to a citrullinated epitope on deiminated human histone 2A and/or histone 4 blocks NET formation. Citrullination of histones is associated with the formation of NETs. Blocking of NET formation can be total or partial. For example, the antibody or binding fragment thereof of the invention may reduce NET formation from 10 to 50%, at least 50% or at least 70%, 80%, 90%, 95% or 99%. NET blocking can be measured by any suitable means, for example by measuring NETosis *in vitro* (Kraaij T et al., 2016, Autoimmun. Rev. 15, 577-584).

The antibodies or binding fragments thereof suitable for us in the methods of the invention specifically bind to a citrullinated epitope on deiminated human histone 2A and/or histone 4. The binding of antibodies or binding fragments thereof to a citrullinated epitope on deiminated human histone 2A and/or histone 4 blocks EET formation. Citrullination of histones is associated with the formation of EETs.

Blocking of EET formation can be total or partial. For example, the antibody or binding fragment thereof may reduce EET formation from 10 to 50%, at least 50% or at least 70%, 80%, 90%, 95% or 99%. EET blocking can be measured by any suitable means, for example by measuring EETosis *in vitro* (Fukuchi et al., "How to detect eosinophil ETosis (EETosis) and extracellular traps"; Allergology International, Volume 70, Issue 1, 2021, Pages 19-29).

The terms "binding activity" and "binding affinity" are intended to refer to the tendency of an antibody molecule to bind or not to bind to a target. Binding affinity may be quantified by determining the dissociation constant (Kd) for an antibody and its target. Similarly, the specificity of binding of an antibody to its target may be defined in terms of the comparative dissociation constants (Kd) of the antibody for its target as compared to the dissociation constant with respect to the antibody and another, non-target molecule. Typically, the Kd for the antibody with respect to the target will be 2-fold, preferably 5-fold, more preferably 10-fold less than the Kd with respect to the other, non-target molecule such as unrelated material or accompanying material in the environment. More preferably, the Kd will be 50-fold less, even more preferably 100-fold less, and yet more preferably 200-fold less.

The value of this dissociation constant can be determined directly by well-known methods, and can be computed even for complex mixtures by methods such as those, for example, set forth in Caceci MS and Cacheris WP (1984, Byte, 9, 340-362). For example, the Kd may be established using a double-filter nitrocellulose filter binding assay such as that disclosed by Wong I and Lohman TM (1993, Proc. Natl. Acad. Sci. USA, 90, 5428-5432) or for example, by using Octet surface plasmon resonance.

One method for the evaluation of binding affinity for deiminated human histone 2A and/or histone 4 is by ELISA. Other standard assays to evaluate the binding ability of ligands such as antibodies towards targets are known in the art, including for example, Western blots, RIAs, and flow cytometry analysis. The binding kinetics (e.g. binding affinity) of the antibody also can be assessed by standard assays known in the art, such as surface plasmon resonance, for example by Biacore^{™} system analysis.

Preferably the antibody has a binding affinity for deiminated human histone 2A and/or histone 4 of 1 nM or less. Preferably the antibody of the invention has a binding affinity for deiminated human histone 2A and/or histone 4, and/or deiminated human histone H3 of 0.5 nM or less, 0.1 nM or less, 50 pM or less, 10 pM or less, 5 pM or less, 2 pM or less or 1 pM or less.

The antibody or binding fragment thereof may also be a fusion protein comprising the antigen-binding domain of a native antibody or an aptamer, such as an aptamer in the form of DNA or RNA.

Preferably the antibody or binding fragment thereof of the invention is a monoclonal antibody. Monoclonal antibodies are immunoglobulin molecules that are identical to each other and have a single binding specificity and affinity for a particular epitope. Monoclonal antibodies (mAbs) of the present invention can be produced by a variety of techniques, including conventional monoclonal antibody methodology, for example those disclosed in "Monoclonal Antibodies: a manual of techniques"(Zola H, 1987, CRC Press) and in "Monoclonal Hybridoma Antibodies: techniques and applications" (Hurrell JGR, 1982 CRC Press).

The antibody or binding fragment thereof of the invention comprises a binding domain. A binding domain will generally comprise 6 CDRs (3 in case of VHH), three from a heavy chain and three from a light chain. In one embodiment the CDRs are in a framework and together form a variable region or domain. Thus in one embodiment an antibody or binding fragment comprises a binding domain specific for the antigen comprising a light chain variable region or domain and a heavy chain variable region or domain.

The residues in antibody variable domains are conventionally numbered according to IMGT (http://www.imgt.org). This system is set forth in Lefranc MP (1997, J, Immunol. Today, 18, 509). This numbering system is used in the present specification except where otherwise indicated.

The IMGT residue designations do not always correspond directly with the linear numbering of the amino acid residues. The actual linear amino acid sequence may contain fewer or additional amino acids than in the strict IMGT numbering corresponding to a shortening of, or insertion into, a structural component, whether framework or CDR, of the basic variable domain structure. The correct IMGT numbering of residues may be determined for a given antibody by alignment of residues of homology in the sequence of the antibody with a "standard" IMGT numbered sequence.

The CDRs of the heavy chain variable domain are located at residues 27-38 (CDR1 of VH), residues 56-65 (CDR2 of VH) and residues 105-117 (CDR3 of VH) according to the IMGT numbering system.

The CDRs of the light chain variable domain are located at residues 27-38 (CDR1 of VL), residues 56-65 (CDR2 of VL) and residues 105-117 (CDR3 of VL) according to the IMGT numbering system.

The antibodies or binding fragments thereof of the present invention are set out herein by the primary amino acid sequence of their CDR regions. The antibodies or binding fragments thereof of the present invention are disclosed herein by the primary amino acid sequence of their heavy and light chains.

In particularly preferred embodiment, a modified CDR1 of the VL of an antibody or binding fragment thereof that specifically is employed to provide improved properties to the antibody or binding fragment thereof over an antibody or binding fragment thereof comprising an unmodified version of CDR1 of the VL. In one embodiment, the unmodified CDR1 of the VL of the antibody used to derive such a modified antibody comprises or consists of the amino acid sequences QSLLDSDGKTY (SEQ ID NO: 36) or QSLVDSDGKTY (SEQ ID NO: 37).

The modified CDR1 of the VL chain of the antibody or binding fragment thereof preferably comprises or consists of the amino acid sequence QSL-X₁-D-X₂-D-X₃-KTY, wherein X₁ is V or L, X₂ is T, S, A or N and X₃ is G or A, provided that the amino acid sequence is not QSLLDSDGKTY (SEQ ID NO: 36) or QSLVDSDGKTY (SEQ ID NO: 37). The modified CDR1 of the VL chain of the antibody or binding fragment thereof of shows reduced isomerisation, in comparison with the unmodified CDR1 of SEQ ID NO: 36 or 37, but maintains the binding properties of the unmodified CDR1.

The amino acid sequences of the CDRs for the VH of a particular antibody or binding fragment thereof of the invention are shown in SEQ ID NOs: 1, 2 and 3. The CDRs 2 and 3 for the VL are shown in SEQ ID NOs: 4 and 5.

The amino acid sequences of the VH and VL of a particular antibody or binding fragment thereof of a particularly preferred antibody are given in SEQ ID NOs: 11 and 13. The CDRs for the VH are shown in SEQ ID NOs: 1, 2 and 3. The CDRs for the VL are shown in SEQ ID NOs: 6, 4 and 5.

The amino acid sequences of the VH and VL of another preferred antibody or binding fragment thereof are given in SEQ ID NOs: 11 and 14. The CDRs for the VH are shown in SEQ ID NOs: 1, 2 and 3. The CDRs for the VL are shown in SEQ ID NOs: 7, 4 and 5.

The amino acid sequences of the VH and VL of another preferred antibody or binding fragment thereof are given in SEQ ID NOs: 11 and 15. The CDRs for the VH are shown in SEQ ID NOs: 1, 2 and 3. The CDRs for the VL are shown in SEQ ID NOs: 8, 4 and 5.

The amino acid sequences of the VH and VL of another preferred antibody or binding fragment thereof are given in SEQ ID NOs: 11 and 16. The CDRs for the VH are shown in SEQ ID NOs: 1, 2 and 3. The CDRs for the VL are shown in SEQ ID NOs: 9, 4 and 5.

The amino acid sequences of the VH and VL of another preferred antibody or binding fragment thereof are given in SEQ ID NOs: 11 and 17. The CDRs for the VH are shown in SEQ ID NOs: 1, 2 and 3. The CDRs for the VL are shown in SEQ ID NOs: 10, 4 and 5.

The amino acid sequences of the VH and VL of another preferred antibody or binding fragment are given in SEQ ID NOs: 12 and 13. The CDRs for the VH are shown in SEQ ID NOs: 1, 2 and 3. The CDRs for the VL are shown in SEQ ID NOs: 6, 4 and 5.

The amino acid sequences of the VH and VL of another preferred antibody or binding fragment thereof are given in SEQ ID NOs: 12 and 14. The CDRs for the VH are shown in SEQ ID NOs: 1, 2 and 3. The CDRs for the VL are shown in SEQ ID NOs: 7, 4 and 5.

The amino acid sequences of the VH and VL of another preferred antibody or binding fragment thereof are given in SEQ ID NOs: 12 and 15. The CDRs for the VH are shown in SEQ ID NOs: 1, 2 and 3. The CDRs for the VL chain are shown in SEQ ID NOs: 8, 4 and 5.

The amino acid sequences of the VH and VL of another preferred antibody or binding fragment are given in SEQ ID NOs: 12 and 16. The CDRs for the VH are shown in SEQ ID NOs: 1, 2 and 3. The CDRs for the VL are shown in SEQ ID NOs: 9, 4 and 5.

The amino acid sequences of the VH and VL of another preferred antibody or binding fragment thereof are given in SEQ ID NOs: 12 and 17. The CDRs for the VH are shown in SEQ ID NOs: 1, 2 and 3. The CDRs for the VL are shown in SEQ ID NOs: 10, 4 and 5.

In an embodiment, the antibody of the invention comprises the heavy chain variable domain amino acid sequence of SEQ ID NO: 11, the light chain variable domain amino acid sequence of SEQ ID NO: 16, a heavy chain constant region amino acid sequence comprising SEQ ID NO: 23 or 56, and the light chain constant region amino acid sequence of SEQ ID NO: 24.

In an embodiment, the antibody of the invention comprises the heavy chain variable domain amino acid sequence of SEQ ID NO: 11, the light chain variable domain amino acid sequence of SEQ ID NO: 16, the heavy chain constant region amino acid sequence of SEQ ID NO: 23 or 56, and the light chain constant region amino acid sequence of SEQ ID NO: 24.

An antibody or binding fragment thereof employed in the invention may comprise one or more of the CDR sequences of any one of the specific antibodies as described above, except that the CDR1 of the VL is always present as either comprising or consisting of the amino acid sequence QSL-X₁-D-X₂-D-X₃-KTY, wherein X₁ is V or L, X₂ is T, S, A or N and X₃ is G or A, provided that the amino acid sequence is not QSLLDSDGKTY (SEQ ID NO: 36) or QSLVDSDGKTY (SEQ ID NO: 37), or either comprises or consists of SEQ ID NOs: 6, 7, 8, 9 or 10.

An antibody or binding fragment thereof employed in the invention may comprise one or more VH CDR sequences and alternatively or additionally one or more VL CDR sequences of said specific antibody, in addition to VL CDR1. An antibody or binding fragment thereof may comprise one, two or all three of the VH CDR sequences of a specific antibody or binding fragment thereof as described above and alternatively or additionally one, two or all three of the VL chain CDR sequences of said specific antibody or binding fragment thereof, including VL CDR1. An antibody or binding fragment thereof may comprises all six CDR sequences of a specific antibody or binding fragment as described above. By way of example, an antibody to be employed in the invention may comprise one of SEQ ID NO: 6, 7, 8, 9 or 10 and one or more of SEQ ID NOs: 1, 2, 3, 4 and 5.

In an embodiment of the invention, the modified CDR1 of the VL chain of the antibody or binding fragment thereof of the antibody employed comprises or consists of the amino acid sequence QSL-Z₁-Z₂-Z₃-Z₄-Z₅-KTY, wherein Z₁ is V or L, Z₂ is D or E, Z₃ is T, S, A or N, Z₄ is D, E, S or A and Z₅ is G or A, provided that the amino acid sequence is not QSLLDSDGKTY (SEQ ID NO: 36) or QSLVDSDGKTY (SEQ ID NO: 37). The modified CDR1 of the VL chain of the antibody or binding fragment thereof of the invention shows reduced isomerisation, in comparison with the unmodified CDR1 of SEQ ID NO: 36 or 37, but maintains the binding properties of the unmodified CDR1. The modified CDR1 of the VL chain of the antibody or binding fragment thereof may comprise or consist of in some embodiments SEQ ID NO: 6, 7, 8, 9, 10, 41, 42, 43, 44, 45, 46, 47, 48, 49, 50, 51 or 52. In an embodiment, the antibody may comprise one of SEQ ID NO: 6, 7, 8, 9, 10, 41, 42, 43, 44, 45, 46, 47, 48, 49, 50, 51 or 52, and one or more of SEQ ID NOs: 1, 2, 3, 4 and 5. In one embodiment, the antibody comprises one of SEQ ID NO: 6, 7, 8, 9, 10, 41, 42, 43, 44, 45, 46, 47, 48, 49, 50, 51 or 52, and all of SEQ ID NOs: 1, 2, 3, 4 and 5.

An antibody or binding fragment thereof to be employed in the invention may alternatively comprise a variant of one of these heavy chain variable domains or CDR sequences in CDR2 or 3 of the VL. For example, a variant may be a substitution, deletion or addition variant of any of the above amino acid sequences.

A variant antibody may comprise 1, 2, 3, 4, 5, up to 10, up to 20, up to 30 or more amino acid substitutions and/or deletions from the specific sequences and fragments discussed above, whilst maintaining the activity of the antibodies described herein. "Deletion" variants may comprise the deletion of, for example, 1, 2, 3, 4 or 5 individual amino acids or of one or more small groups of amino acids such as 2, 3, 4 or 5 amino acids. "Small groups of amino acids" can be defined as being sequential, or in close proximity but not sequential, to each other. "Substitution" variants preferably involve the replacement of one or more amino acids with the same number of amino acids and making conservative amino acid substitutions. For example, an amino acid may be substituted with an alternative amino acid having similar properties, for example, another basic amino acid, another acidic amino acid, another neutral amino acid, another charged amino acid, another hydrophilic amino acid, another hydrophobic amino acid, another polar amino acid, another aromatic amino acid, another aliphatic amino acid, another tiny amino acid, another small amino acid or another large amino acid. Some properties of the 20 main amino acids, which can be used to select suitable substituents, are as follows:

| | | | |
|---|---|---|---|
| Ala | aliphatic, hydrophobic, neutral | Met | hydrophobic, neutral |
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gln | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, charged (+) | Thr | polar, hydrophilic, neutral |
| Ile | aliphatic, hydrophobic, neutral | Val | aliphatic, hydrophobic, neutral |
| Lys | polar, hydrophilic, charged (+) | Trp | aromatic, hydrophobic, neutral |
| Leu | aliphatic, hydrophobic, neutral | Tyr | aromatic, polar, hydrophobic |

Preferred "derivatives" or "variants" include those in which instead of the naturally occurring amino acid the amino acid, which appears in the sequence, is a structural analog thereof. Amino acids used in the sequences may also be derivatized or modified, e.g. labelled, providing the function of the antibody is not significantly adversely affected.

Derivatives and variants as described above may be prepared during synthesis of the antibody or by post-production modification, or when the antibody is in recombinant form using the known techniques of site-directed mutagenesis, random mutagenesis, or enzymatic cleavage and/or ligation of nucleic acids.

Preferably variant antibodies have an amino acid sequence which has more than 60%, or more than 70%, e.g. 75 or 80%, preferably more than 85%, e.g. more than 90%, 95%, 96%, 97%, 98% or 99% amino acid identity to the VL and/or VH, or a fragment thereof, of an antibody disclosed herein. This level of amino acid identity may be seen across the full-length of the relevant SEQ ID NO sequence or over a part of the sequence, such as across 20, 30, 50, 75, 100, 150, 200 or more amino acids, depending on the size of the full-length polypeptide.

Preferably the variant antibodies comprise one or more of the CDR sequences as described herein.

In connection with amino acid sequences, "sequence identity" refers to sequences, which have the stated value when assessed using ClustalW (Thompson JD et al., 1994, Nucleic Acid Res., 22, 4673-4680) with the following parameters:
Pairwise alignment parameters -Method: slow/accurate, Matrix: PAM, Gap open penalty: 10.00, Gap extension penalty: 0.10;
Multiple alignment parameters -Matrix: PAM, Gap open penalty: 10.00, % identity for delay: 30, Penalize end gaps: on, Gap separation distance: 0, Negative matrix: no, Gap extension penalty: 0.20, Residue-specific gap penalties: on, Hydrophilic gap penalties: on, Hydrophilic residues: G, P, S, N, D, Q, E, K, R. Sequence identity at a particular residue is intended to include identical residues, which have simply been derivatized.

Thus, antibodies having specific VH and VL amino acid sequences and variants and fragments thereof, which maintain the function or activity of these VHs and VLs, may be employed in the present invention.

Accordingly, antibodies or binding fragments thereof may be employed comprising variants of the VH that retain the ability of specifically binding a citrullinated epitope on human deiminated human histone 2A and/or histone 4. A variant of the heavy chain may have at least 70%, at least 80%, at least 85%, at least 90%, at least 95%, at least 96%, at least 97%, at least 98% or at least 99% amino acid sequence identity to the unmodified VH. The variant of the VH may comprise a fragment of at least 7 amino acids of hVH22. 101f or hVH22.101HC9 (SEQ ID NO: 11 and 12, respectively), wherein the antibody or binding fragment thereof retains the ability of being specifically reactive with a citrullinated epitope on deiminated human histone 2A and/or histone 4; or a variant of hVH22.101f or hVH22.101HC9 (SEQ ID NO: 11 and 12, respectively) having at least 70% amino acid sequence identity to a sequence of hVH22.101f or hVH22.101HC9 (SEQ ID NO: 11 and 12, respectively), wherein the antibody or binding fragment thereof retains the ability of being specifically reactive with a citrullinated epitope on deiminated human histone 2A and/or histone 4.

### Polynucleotides, vectors and host cells

Polynucleotides, vectors and expression vectors encoding the antibody or binding fragments thereof described herein, particularly by way of providing illustrative guidance as to how antibodies and binding fragments of the present invention may be produced, though they may be administered in some embodiments, provided they result in the stated dose of antibody of binding fragment thereof being administered.

A polynucleotide may encode any antibody or fragment as described herein. The terms "nucleic acid molecule" and "polynucleotide" are used interchangeably herein and refer to a polymeric form of nucleotides of any length, either deoxyribonucleotides or ribonucleotides, or analogs thereof. Non-limiting examples of polynucleotides include a gene, a gene fragment, messenger RNA (mRNA), cDNA, genomic DNA, recombinant polynucleotides, plasmids, vectors, isolated DNA of any sequence, isolated RNA of any sequence, nucleic acid probes, and primers. A polynucleotide may be provided in isolated or purified form.

A nucleic acid sequence which "encodes" a selected polypeptide is a nucleic acid molecule, which is transcribed (in the case of DNA) and translated (in the case of mRNA) into a polypeptide *in vivo* when placed under the control of appropriate regulatory sequences. The boundaries of the coding sequence are determined by a start codon at the 5' (amino) terminus and a translation stop codon at the 3' (carboxy) terminus. For the purposes of the invention, such nucleic acid sequences can include, but are not limited to, cDNA from viral, prokaryotic or eukaryotic mRNA, genomic sequences from viral or prokaryotic DNA or RNA, and even synthetic DNA sequences. A transcription termination sequence may be located 3' to the coding sequence. In one embodiment, a polynucleotide comprises a sequence, which encodes a VH or VL amino acid sequence as described above. The polynucleotide may encode the VH or VL sequence of a specific antibody or binding fragment thereof as disclosed herein.

An antibody or binding fragment thereof of the invention may thus be produced from or administered to a patient in the form of a polynucleotide, which encodes, and is capable of expressing it. Where the antibody comprises two or more chains, a polynucleotide of the invention may encode one or more antibody chains. For example, a polynucleotide may encode an antibody light chain, an antibody heavy chain or both. Two polynucleotides may be provided, one of which encodes an antibody light chain and the other of which encodes the corresponding antibody heavy chain. Such a polynucleotide or pair of polynucleotides may be expressed together such that an antibody of the invention is generated.

Polynucleotides of the invention can be synthesised according to methods well known in the art, as described by way of example in Sambrook J et al. (1989, Molecular cloning: a laboratory manual; Cold Spring Harbor: New York: Cold Spring Harbor Laboratory Press).

The nucleic acid molecules may be provided in the form of an expression cassette, which includes control sequences operably linked to the inserted sequence, thus allowing for expression of the antibody of the invention *in vivo.* These expression cassettes, in turn, are typically provided within vectors (e.g., plasmids or recombinant viral vectors). Such an expression cassette may be administered directly to a host subject. Alternatively, a vector comprising a polynucleotide of the invention may be administered to a host subject. Preferably the polynucleotide is prepared and/or administered using a genetic vector. A suitable vector may be any vector, which is capable of carrying a sufficient amount of genetic information, and allowing expression of a polypeptide of the invention.

Expression vectors that comprise such polynucleotide sequences may be employed. Such expression vectors are routinely constructed in the art of molecular biology and may for example involve the use of plasmid DNA and appropriate initiators, promoters, enhancers and other elements, such as for example polyadenylation signals, which may be necessary, and which are positioned in the correct orientation, in order to allow for expression. Other suitable vectors would be apparent to persons skilled in the art. By way of further example in this regard we refer to Sambrook J et al. (1989, Molecular cloning: a laboratory manual; Cold Spring Harbor: New York: Cold Spring Harbor Laboratory Press).

A person skilled in the art may use the sequences described herein to clone or generate cDNA or genomic sequences for instance such as described in the below examples. Cloning of these sequences in an appropriate eukaryotic expression vector, like pcDNA3 (Invitrogen), or derivatives thereof, and subsequent transfection of mammalian cells (like CHO cells) with combinations of the appropriate light and heavy chain-containing vectors will result in the expression and secretion of the antibodies described herein.

The skilled person may also make analogues of the antibodies or binding fragments thereof as described herein by using the specific binding domains of the antibody sequences and express them in a different context, such as a polypeptide, such as a fusion protein. This is well known in the art.

Cells that have been modified to express an antibody of the invention may also be employed. Such cells include transient, or preferably stable higher eukaryotic cell lines, such as mammalian cells or insect cells, lower eukaryotic cells, such as yeast or prokaryotic cells, such as bacterial cells. Particular examples of cells, which may be modified by insertion of vectors or expression cassettes encoding for an antibody of the invention, include mammalian HEK293, CHO, HeLa, NS0 and COS cells. Preferably the cell line selected will be one which is not only stable, but also allows for mature glycosylation.

Such cell lines may be cultured using routine methods to produce an antibody or binding fragment thereof of the invention, or may be used therapeutically or prophylactically to deliver antibodies or binding fragments thereof of the invention to a subj ect.

### Pharmaceutical compositions

The antibodies or binding fragments thereof may be in the form of a pharmaceutical composition comprising the antibodies or binding fragments thereof and a pharmaceutically acceptable carrier.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for parenteral, e.g. intravenous, intraocular, intramuscular, subcutaneous, intradermal or intraperitoneal administration (e.g. by injection or infusion). In certain embodiments, a pharmaceutically acceptable carrier comprises at least one carrier selected from the group consisting of a co-solvent solution, liposomes, micelles, liquid crystals, nanocrystals, nanoparticles, emulsions, microparticles, microspheres, nanospheres, nanocapsules, polymers or polymeric carriers, surfactants, suspending agents, complexing agents such as cyclodextrins or adsorbing molecules such as albumin, surface active particles, and chelating agents. In further embodiments, a polysaccharide comprises hyaluronic acid and derivatives thereof, dextran and derivatives thereof, cellulose and derivatives thereof (e.g. methylcellulose, hydroxy-propylcellulose, hydroxypropylmethylcellulose, carboxymethylcellulose, cellulose acetate phthalate, cellulose acetate succinate, cellulose acetate butyrate, hydroxypropylmethyl-cellulose phthalate), chitosan and derivative thereof, [beta]-glucan, arabinoxylans, carrageenans, pectin, glycogen, fucoidan, chondrotin, dermatan, heparan, heparin, pentosan, keratan, alginate, cyclodextrins, and salts and derivatives, including esters and sulfates, thereof.

Preferred pharmaceutically acceptable carriers comprise aqueous carriers or diluents. Examples of suitable aqueous carriers that may be employed in the pharmaceutical compositions include water, buffered water and saline. Examples of other carriers include ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants. In many cases, it will be preferable to include isotonic agents, for example, sugars, polyalcohols, such as mannitol, sorbitol, or sodium chloride in the composition.

A pharmaceutical composition also may include a pharmaceutically acceptable anti-oxidant. These compositions may also contain adjuvants, such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents, which delay absorption such as aluminium monostearate and gelatin.

Therapeutic compositions typically must be sterile and stable under the conditions of manufacture and storage. The pharmaceutical composition can be formulated as a solution, microemulsion, liposome, or other ordered structure suitable to high drug concentration.

In further embodiments, the pharmaceutical compositions described herein can be administered by a route such as intravenous, subcutaneous, intraocular, intramuscular, intra-articular, intradermal, intraperitoneal, spinal or by other parenteral routes of administration, for example by injection or infusion. Administration may be rectal, oral, ocular, topical, epidermal or by the mucosal route. Administration may be local, including peritumoral, juxtatumoral, intratumoral, to the resection margin of tumors, intralesional, perilesional, by intra cavity infusion, intravesicle administration, or by inhalation. In a particularly preferred embodiment, the pharmaceutical composition is administered intravenously or subcutaneously. Hence, in one particularly preferred embodiment, the route of administration is intravenous. In another particularly preferred embodiment, the route of administration is subcutaneous. Subcutaneous injection is an especially preferred route. Intravenous administration by infusion or injection is also especially preferred as an administration route.

In one preferred embodiment, the antibody or fragment is provided in a pharmaceutical composition specifically formulated for injection. In one preferred embodiment, the antibody or fragment is provided in a pharmaceutical composition specifically formulated for subcutaneous injection. In one preferred embodiment, the antibody or fragment is provided in a pharmaceutical composition specifically formulated intravenous injection.

Sterile injectable solutions can be prepared by incorporating the active agent (e.g. antibody) in the required amount in an appropriate solvent with one or a combination of ingredients enumerated above, as required, followed by sterilization microfiltration. Generally, dispersions are prepared by incorporating the active agent into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active agent plus any additional desired ingredient from a previously sterile-filtered solution thereof.

The pharmaceutical composition employed may be presented in a preloaded device, for example it may be present in a preloaded syringe. In one embodiment, the pharmaceutical composition is in the form of an i.v. bag comprising the pharmaceutical composition. In one embodiment, the pharmaceutical composition is present in a vial.

Dosage unit form as used herein refers to physically discrete units suited as unitary dosages for the subjects to be treated; each unit contains a predetermined quantity of active compound calculated to produce the desired therapeutic effect in association with the required pharmaceutical carrier. In one preferred embodiment a preloaded device, i.v. bag, or vial is in unit dosage form.

Pharmaceutical compositions may comprise additional active ingredients as well as an antibody. As mentioned above, compositions may comprise one or more antibodies of the invention. They may also comprise additional therapeutic or prophylactic active agents. Such additional agents may be conjugated to the antibody.

Also within the scope of the present invention are kits comprising antibodies or other compositions of the invention and instructions for use. The kit may further contain one or more additional reagents, such as an additional therapeutic or prophylactic agent as discussed herein.

The present invention is further illustrated by the following examples which should not be construed as further limiting. The contents of all figures and all references, patents and published patent applications cited throughout this application are expressly incorporated herein by reference.

### Examples

### Example 1

### Introduction

A first-in human, randomized, double-blind, randomized, placebo-controlled, single ascending dose study was performed involving administration of CIT-013, which is a particularly preferred example of an antibody or binding fragment thereof that specifically binds to a citrullinated epitope on deiminated human histones. The study was performed in healthy volunteers with intravenous lipopolysaccharide (LPS) challenge which stimulates an inflammatory response, including the production of NETs from neutrophils. Objectives of the LPS challenge in which healthy subjects have been dosed with CIT-013 followed by LPS included the following: (i) safety and tolerability; (ii) antibody pharmokinetics; and (iii) pharmacodynamic effects of antibody administration by characterising the inflammatory response. Analysis of (i) to (iii) helped in the identification of preferred dosages of antibody for inhibition of NET formation and hence treatment of NET associated pathologies. The study was a single centre study.

### Overall study design and plan

Table 2 gives the study setup for the group given a 0.3 mg/kg of antibody prior to LPS challenge. Table 3 gives the study setup for the group given a 0.9 mg/kg of antibody prior to intravenous LPS challenge.

The total duration of the study for each subject was up to 131 days, divided as follows:
▪ Screening: Up to 42 days before dosing;
▪ Treatment and study assessments: Days -1 to 84
▪ In Clinic period: Days -1 to 2;
▪ Follow-up visit: 79 to 89 days after last dose.
Subjects were admitted to the study unit on Day -1 and discharged approximately 36 hours after study drug administration.

### Subjects/Groups

Cohort 1a consisted of 1 active and 2 placebo subjects, with the active subjects administered 0.3 mg/kg of antibody intravenously. Cohort 1b consisted of 2 active and 1 placebo subjects 0.3 mg/kg of antibody intravenously. Cohort 2 consisted of 8 active and 6 placebo subjects, with the active subjects administered 0.9 mg/kg.

### Inclusion criteria

All eligible subjects for the study had to meet the following criteria at screening:
1. Healthy men or women, 18 to 55 years of age (inclusive) at screening. For cohort 2 only healthy men were included. The health status verified by absence of evidence of any clinically significant active or uncontrolled chronic disease following a detailed medical history, a complete physical examination including vital signs, laboratory measurements, and 12-lead ECG;
2. Signed informed consent, able and willing to comply with the requirements of the study protocol.
3. Body mass index (BMI) between 18 and 32 kg/m², inclusive, and a body weight between 50 and 150 kg, inclusive at screening.
4. All male and Women of Child Bearing Potential volunteers had to practice effective contraception during the study and be willing and able to continue contraception for at least 90 days after their last dose of study treatment.
5. Ability to communicate well with the Investigator in the Dutch language and willing to comply with the study restrictions.

### Exclusion criteria

Subjects were excluded if they met any of the following criteria at screening or pre-dose:
1. Evidence (following a detailed medical history, physical examination, vital signs, 12-lead ECG and clinical laboratory parameters) of any active or chronic disease or condition that could interfere with, or for which the treatment might interfere with, the conduct of the study, or that would pose an unacceptable risk to the subj ect in the opinion of the investigator.
2. Clinically significant abnormalities, as judged by the investigator, in laboratory test results (including hepatic and renal panels, complete blood count, chemistry panel and urinalysis). Minor deviations of laboratory values from the normal range may be accepted, if judged by the Investigator or medically qualified designee as not clinically significant. In the case of uncertain or questionable results, tests performed during screening may be repeated before randomization to confirm eligibility or judged to be clinically irrelevant for healthy subjects.
3. Any confirmed or suspected disease or condition associated with immune system impairment, including auto-immune diseases, HIV, asplenia or recurrent severe infections.
4. Use of chronic (more than 14 days) immunosuppressant or immunomodulatory drugs within the 3 months prior to IMP administration, or isolated (non-chronic) use within 30 days prior to IMP administration.
5. Any history of severe allergic reaction(s).
6. Any confirmed significant drug hypersensitivity reactions (including skin reactions or anaphylaxis), or known allergies (non-active hay fever is acceptable).
7. Positive Hepatitis B surface antigen (HBsAg), Hepatitis C antibody (HCV Ab), or human immunodeficiency virus antibody (HIV Ab) at screening, or other known infection requiring systemic antibiotic therapy within three months prior to the study.
8. Subject having an active, uncontrolled acute or chronic systemic fungal, bacterial, and/or viral, infection within the past 30 days.
9. Subjects with evidence or history of clinically significant haematological, renal, endocrine, pulmonary, gastrointestinal, cardiovascular, hepatic, psychiatric, neurologic diseases.
10. Subjects with a positive urine drug screen at screening or pre-dose.
11. Subject with a positive SARS-CoV-2 PCR based test within 72 hours prior to receiving CIT-013.
12. History of abuse of addictive substances (alcohol, illegal substances) or current use of more than 14 units alcohol per week, drug abuse, or regular user of sedatives, hypnotics, tranquillisers, or any other addictive agent,
13. Treatment with an investigational drug within 30 days or 5 half-lives (whichever is longer) preceding the first dose of CIT-013.
14. Use of prescription or over-the-counter (OTC) drugs, vitamins, minerals and dietary supplements, within 7 days or 5 half-lives (whichever is longer) prior to the first dose of study medication until End of Study (EOS). Herbal supplements and hormone replacement therapy must be discontinued 30 days prior to the first dose of study medication until EOS. Excluded from this list is paracetamol at doses of <4 g/day on all study days except day 1 of part B. Exceptions were only made if the rationale was clearly documented by the investigator.
15. Receipt of live or attenuated vaccine 90 days prior to first study intervention administration.
16. Vaccination (completion of 2nd vaccination shot if applicable) against SARS-CoV-2 or influenza vaccinations less than 14 days prior to first study drug administration.
17. Known hypersensitivity to any of the constituents or excipients of CIT-013 or history of relevant drug and/or food allergy (anaphylactic, anaphylactoid reactions).
18. Excessive caffeine consumption, defined as >800 mg per day from 7 days prior to the first dose of the study drug until 24 hours prior to dosing. Subjects to abstain from caffeine-containing products for 24 hours prior to the start of dosing until discharge from the study unit. Caffeine quantities defined as: one cup of coffee contains 100 mg of caffeine; one cup of tea, or one glass of cola, or portion of chocolate (dark: 100 g, milk 200 g) contains approximately 40 mg of caffeine; one bottle of Red Bull contains approximately 80 mg of caffeine.
19. Donation (or loss) of whole blood or plasma of 500 mL or more during the 12 weeks prior to CIT-013 administration.
20. Smoking > than 10 cigarettes (or equivalent) per week and/or using nicotine-based products within 1 month prior to CIT-013 administration and/or unwillingness to abstain from the use of these from screening until EOS.
21. Any other known factor, condition, or disease that, in the opinion of the Investigator, might interfere with treatment compliance, study conduct or interpretation of the results, or may compromise volunteer safety.
22. Extreme exercise (e.g. marathon or triathlon) within 2 weeks of screening.
23. Participation of the subject in an intravenous LPS challenge study before.

### Concomitant medications

Subjects also had to follow the below regarding concomitant medicines:
- No prescription or over-the-counter (OTC) drugs, vitamins, minerals and dietary supplements, were permitted within 7 days or 5 half-lives (whichever is longer) prior to the first dose of study medication until EOS. Herbal supplements and hormone replacement therapy to be discontinued 30 days prior to the first dose of study medication until EOS. Excluded from the list is paracetamol at doses of <4 g/day on all study days except day 1. Exceptions were only to be made if the rationale was clearly documented by the investigator.
- No chronic (more than 14 days) immunosuppressant or immunomodulatory drugs within the 3 months prior to IMP administration, or isolated (non-chronic) use within 30 days prior to IMP administration.
- Based on the observed adverse event profile in the previous conducted single ascending dose study in healthy volunteers, which are considered most likely to be of gastrointestinal origin, it was decided to allow pre-dose medications.
- All medications (prescription and over-the-counter [OTC]) taken within 30 days of study screening until the final end-of-study follow-up visit will be recorded in the CRF.

### Sample size justification

For the sample size, a formal power calculation was performed on NET in plasma as endpoint which indicated that 8 subjects per study group would provide a power of 80% to detect 40% reduction in LPS-induced NET components (MPO: Myeloperoxidase) by CIT-013, assuming a common standard deviation of 0.0503 and a significancy level of 0.05 using two-sample t-test. All group comparisons were conducted versus the placebo group of 6 subj ects.

Sentinel dosing was applied in the 3 subjects of LPS cohort 1a. Those subjects were dosed in a blinded randomized way (1 active, 2 placebo). The first 3 subjects of that cohort were observed and monitored (vital signs, physical examination findings, ECG and laboratory parameters) for at least 48 hours for detection of any acute, adverse events. After this period the remaining 3 subjects of cohort 1b were dosed. All of the subjects in both cohorts 1a and 1b apart from those administered the placebo were given 0.3 mg/kg

### Screening

The Screening Visit was conducted anytime up to 42 days prior to Baseline/admission to CHDR. The screening phase only started after full written, verbal and signed informed consent has been obtained, according to CHDR standard operating procedures. A full medical screening was performed to assess a subject's eligibility for the study. The overview of the assessments of this visit is provided in the study flow chart (Table 2 and 3).

### Rescreening

Selected participants were rescreened, if the reason for non-eligibility was considered transient (e.g., abnormal laboratory test, insufficient washout period of a forbidden medication, positive drug screen, etc.). During a re-screening, only assessments that are susceptible to change within the timeframe between screenings are repeated (e.g. medical history, demographics, virology are not repeated).

### Treatment and observation period

The overview of all assessments including time schedule is provided in the study flow charts (Table 2 and 3). Subjects presented to the CRU on Day -1 (i.e., one day prior to study drug administration) for baseline procedures conducted prior to dosing on Day 1. Subjects began overnight fasting period at least 8 hours prior to the morning i.v. dose of CIT-013. Water was allowed *ad libitum.* Subjects stayed overnight at CHDR and were discharged the afternoon of Day 2, approximately 36 hours post-dose.

### Follow-up

Follow-up occurred up until 79 and 89 days after the last study drug administration. The overview of the assessments of that visit is provided in the study flow charts (Table 2 and 3). End of Study (EOS) was defined as the last visit of the last subject. Upon early termination, the follow-up visit was also performed between 21 and 28 days after the last study drug administration.

### Dose selection

In cohorts 1a and 1b the dosage given was 0.3 mg/kg CIT-013 to those receiving the drug rather than the placebo. In cohort 2 the dosage given was 0.9 mg/kg CIT-013 to those receiving the drug rather than the placebo.

### Antibody and matching placebo

The study drug, CIT-013 is an IgG1k monoclonal antibody consisting of two identical light chain polypeptides composed of 219 amino acids each and two identical heavy chain polypeptides composed of 451 amino acids each. Study drug or placebo was administered to the subjects as detailed in (Table 2 and 3). One strength of 250 mg lyophilized CIT-013 per vial has been manufactured and required reconstitution prior to addition to an intravenous (i.v,) administration system. The study drug was administered using the Braunn space P infusion pump at a constant rate over two hours following an eight hour period of fasting. One group given the study drug was administered 0.3 mg/kg, whilst the other was given 0.9 mg.

Water was allowed *ad libitum.* The placebo drug was sodium chloride, 0.9%.

### Blinding

The study was performed in a double-blind fashion. With the exceptions described in this section, the randomization list was not available to the investigator, study staff, subjects, sponsor, medical monitor, or monitors during the study. The investigational drug and its matching placebo were indistinguishable and were packaged in the same way.

The randomization list was only made available to the pharmacist preparing the study drug, to the individual responsible for PK and PD sample bioanalysis and to statisticians involved in preparing blinded summaries, graphs and listings to support the dose decisions. The summaries, graphs and listings provided by the statisticians or programmers were produced in an area to which other team members did not have access. The investigator received a set of sealed randomization codes to be broken in case of emergency situations and duplicates were kept by the unblinded monitor and unblinded medical monitor. If the identity of the study drug administered needed to be known in order to manage the subject's condition i.e., in case of a medical emergency or in the case a SAE occurs, the treatment emergency code for that subject could be broken and the study drug identified. Any such occurrences were documented in the study file. Treatment emergency codes were not to be broken except in emergency situations described above and, if possible, the sponsor was to be contacted before the emergency code was opened. At the final monitoring visit the unused emergency code labels were checked and a statement to the effect that all are intact (or not as the case may be) made on the database lock form.

### Specific safety and tolerability assessments

*Vital signs:* Evaluations of systolic and diastolic blood pressure, pulse rate, and temperature were performed throughout the study. Pulse and blood pressure taken after 5 minutes in the supine position. Automated oscillometric blood pressures and pulse rate were measured using a Dash 3000, Dash 4000, Dynamap 400 or Dynamap ProCare 400.

*Weight and height:* Weight (kg) was recorded at screening, at day -1 and the follow-up visit or upon early termination. Height (cm) was recorded and body mass index (BMI) calculated at screening.

*Physical examination:* Physical examination (i.e., inspection, percussion, palpation and auscultation) was performed during the course of the study. Clinically relevant findings that were present prior to study drug initiation were recorded with the subject's Medical History. Clinically relevant findings found after study drug initiation and meeting the definition of an AE (new AE or worsening of previously existing condition) were recorded.

*Electrocardiography:* ECGs were obtained during the course of the study using Marquette 2000/5500 and stored using the MUSE Cardiology Information. ECGs were taken after at least 5 minutes in the supine position. When timings coincide, ECGs were performed before safety blood sampling. PK sampling took precedence. The investigator assessed the ECG recording as 'normal', 'abnormal - not clinically significant', or 'abnormal - clinically significant' and included a description of the abnormality as required. The ECG parameters assessed included heart rate, PR, QRS, QT, and QTcF (calculated using Fredericia's method).

### Laboratory parameters

Blood and other biological samples were collected for the following clinical laboratory tests, kept at room temperature, and analysed within 4 hours after collection using the methodology set out in Table 1 part A below. Biomarkers mentioned in Table 1 part B were assessed when appropriate number of samples were collected. Table 1 part C shows the type of blood collection tubes and the volumes of blood needed for the various laboratory tests and biomarker assessments.

**Table 1, Part A**

| **Lab** | **Tests** | **Collection & Analysis*** |
|---|---|---|
| Haematology | Haemoglobin [including Mean Corpuscular volume (MCV), Mean corpuscular haemoglobin (MCH), Mean corpuscular haemoglobin concentration (MCHC)], haematocrit, red cell count (RBC), total white cell count (WBC) and Platelet count. Differential blood count, including: basophils, eosinophils, neutrophils, lymphocytes, and monocytes. | 2 mL of venous blood in a BD Vacutainer^{®} K2EDTA tube. Samples will be analysed by the Clinical Chemistry Laboratory (AKCL) of Leiden University Medical Center (LLTMC). |
| Chemistry and electrolytes | Sodium, potassium, calcium, inorganic phosphate, total protein, albumin, triglycerides, blood urea nitrogen (BUN), creatinine, uric acid, total bilirubin², alkaline phosphatase, AST, ALT, gamma-GT, cholesterol ,CRP and LDH. | 3.5 mL of venous blood in a BD Vacutainer^{®} SST Gel and Clot Activator tube. Samples will be analysed by the AKCL of LUMC |
| Glucose | Glucose¹ | 2 mL of venous blood in a BD Vacutainer^{®} Sodium Fluoride tube. Samples will be analysed by the AKCL of LLTMC |
| Serology | HIV1 and HIV2 antigen and antibodies, Hepatitis B surface antigen, Hepatitis B antibodies and Hepatitis C antibodies | 5 mL of venous blood in a BD Vacutainer^{®} SST Gel and Clot Activator tube. Samples will be analysed by the Microbiology Laboratory (CKML) of the LUMC |
| Urinalysis | Leucocytes, blood, nitrite, protein, urobilinogen, bilirubin, pH, specific gravity, ketones, glucose. If there is a clinically significant positive result, urine will be sent to the AKCL for microscopy and/or culture (part A and B) or sediment will be performed according to the local site standard procedure (part C). | A midstream, clean-catch urine specimen will be analysed by dipstick (Multistix^{®} 10 SG, Siemens Healthcare Diagnostics, Frimley, UK). |
| Pregnancy³ | hCG. If there is a clinically significant, positive result, urine will be sent to the AKCL for confirmation (part A and B) or urine will be sent for confirmation according to the local site standard procedure (part C). | A urine specimen will be analysed at CHDR by test kit (InstAlert, Innovacon, San Diego, USA). |
| Alcohol | Alcohol Breath Test | The hand-held Alco-Sensor IV meter (Honac, Apeldoorn, the Netherlands) will be used to measure the breath ethanol concentrations. |
| Urine drug screen | Cocaine, amphetamines, opiates (morphine), benzodiazepines and cannabinoids. | A urine specimen will be analysed at CHDR by test kit (InstAlert, Innovacon, San Diego, USA). |
| ¹ After 4-hours fasting. ²Conjugated bilirubin will be reported only when total bilirubin is outside the reference range. ³Pregnancy test for women will be performed at screening and baseline and if pregnancy is suspected during the study. | | |

| Biomarker Naming | Assay ID | | Catalog ue Number | Vendor |
|---|---|---|---|---|
| Calprotectin | Calprotectin (S100A8/S100A9) | | DS8900 | R&D Systems (Minneapolis; USA) |
| Cathelicidin (LL-37) | Human LL37 / Cathelicidin ELISA | | LS-F31974 | LifeSpan Biosciences, Inc. (Seattle, USA) |
| D-Dimer | Asserachrom D-Di | | 00947 | Stago BNL (Leiden; NL) |
| Myeloperoxidase (MPO) | Human Myeloperoxidase Immunoassay Quantikine^{™} ELISA | | DMYE00B | R&D Systems (Minneapolis; USA) |
| Neutrophil Elastase (HNE) | Human Neutrophil Elastase Simple Step ELISA | | ab270204 | Abeam (Cambridge; UK) |
| Prothrombin fragment F1+2 | Enzygnost Anti-Human F1+F2 | | 64724 | Siemens Healthcare Diagnostics Products GmbH (Marburg, Germany) |

| ***V-PLEX Plus Neuroinflammation Panel 1 Human Kit (consist of three components)*** | | | | |
|---|---|---|---|---|
| TNF-a, IL-1B, IL-6, IL-8 and IL-10 | Proinflammatory Panel 1 | | K15210G | Meso Scale Discovery (Rockville, US |
| CXCL-10 (IP-10) | Chemokine Panel 1 | | K15210G | Meso Scale Discovery (Rockville, USA) |
| VCAM-1, ICAM-1 | Vascular Injury Panel 2 | | K15210G | Meso Scale Discovery (Rockville, USA) |
| | | | | |

| ***Biomarkers analyzed for LPS cohort #2 only*** | | | | |
|---|---|---|---|---|
| Biomarker Naming | Assay ID | | Catalog e Number u | Vendor |
| IL-1 receptor antagonist (IL-1 ra) | V-PLEX Plus Human IL-1RA | | K151WTG | Meso Scale Discovery (Rockville, USA) |
| Plasminogen Activator Inhibitor (PAI-1) | Human Serpin E1/PAI-1 Quantikine ELISA | | DSE100 | R&D Systems (Minneapolis; USA) |
| Plasmin-Antiplasmin (PAP) complex | 1 | HUMAN PLASMIN-ANTIPLASMIN COMPLEX, PAP ELISA | NBP3-06902 | Novus Biologicals, BioTechne Ltd. (Abingdon, United Kingdom) |
| von Willebrandt Factor (vWF) | 2 | HUMAN VWF CALIBRATOR | C01C9-2 | Meso Scale Discovery (Rockville, USA) |
| Tissue Plasminogen Activator (tPA) | 3 | HUMAN T-PLASMINOGEN ACTIVATOR/TPA QUANTIKINE ELISA | DTPA00 | R&D Systems (Minneapolis; USA) |

**Table 1, Part B**

| ***Biomarkers analyzed LPS cohort* #*1 and* #*2 (dsDNA and Nucleosome not included)*** | | | | |
|---|---|---|---|---|
| ***All Assays have been qualified* @ *Ardena*** | | | | |
| ***CitH3 ELISA has been used for in-house LPS sample analysis*** | | | | |
| Biomarker Naming | Assay ID | | Catalogue Number | Vendor |
| Citrullinated Histone H3 (CitH3) | 4 | CITRULLINATED HISTONE H3 (CLONE 1103) ELISA | 501620 | Cayman Chemical (Ann Arbor; USA) |

**Table 1, Part C**

| **Sample** | **Samples taken** | | | **Sample Volume*** | | | **Volume** | |
|---|---|---|---|---|---|---|---|---|
| Haematology (2 mL K2EDTA) | 17 | | x | 2 | mL | = | 34 | mL |
| Chemistry (3.5 mL SST Gel and Clot) | 17 | | x | 3.5 | mL | = | 59.5 | mL |
| Glucose (2 mL Sodium Fluoride) | 3 | | x | 2 | mL | = | 6 | mL |
| ADA (3.5 mL SST tubes) | 6 | | x | 3.5 | mL | = | 21 | mL |
| Serology (5 mL SST Gel and Clot) | 1 | | x | 5 | mL | = | 5 | mL |
| Pharmacokinetics (3.5 mL SST tubes) | 15 | | x | 3.5 | mL | = | 52.5 | mL |
| PD ELISA (3 mL K2EDTA anticoagulant tubes) | 9 | | x | 3 | mL | = | 27 | mL |
| PD NET components (3.5 mL SST) | 15 | | x | 6 | mL | = | 90 | mL |
| PD soluble markers (6 mL K2EDTA anticoagulant tubes) | 15 | | x | 6 | mL | = | 90 | mL |
| PD blood sample Flow cytometry | 4 | | x | 4 | mL | = | 16 | mL |
| * exclusive discarded volume | | | **Total blood volume/subject** | | | | **401** | **mL** |

Clinically relevant findings found of moderate or higher intensity and meeting the definition of an AE (new AE or worsening of previously existing condition) will be recorded as an AE.

### Stopping criteria

The criteria for stopping dose escalation temporarily or for adjustment were:
- in the case of any unacceptable tolerability profile based on the nature, frequency, and intensity of observed AEs, judged jointly by the Principal Investigator, the medical responsible person and the Sponsor's medical monitor.
- if two or more subjects on active drug experienced a serious adverse event (SAE) or severe AEs, which in the opinion of the Principal Investigator and Medical Responsible person or the Sponsor's medical representative would likely to be causally related to IMP.

To make a decision relating to dose increase those making the decision were able to access unblinded data at that point. If confirmed by review of the data that one of these criteria were met and association with drug administration was likely, dose-escalation was to be stopped. That though did not occur.

### Treatment duration

Single doses were administered to achieve the study objectives (safety, tolerability, pharmacokinetics of a single dose of CIT-013). To be able to evaluate the pharmacodynamic effects of CIT-013 in the study, intravenous LPS was administered 2.5 hours after the start of infusion of CIT-013.

### Primary endpoint

There was no formal primary endpoint in the study. All endpoints are considered exploratory.

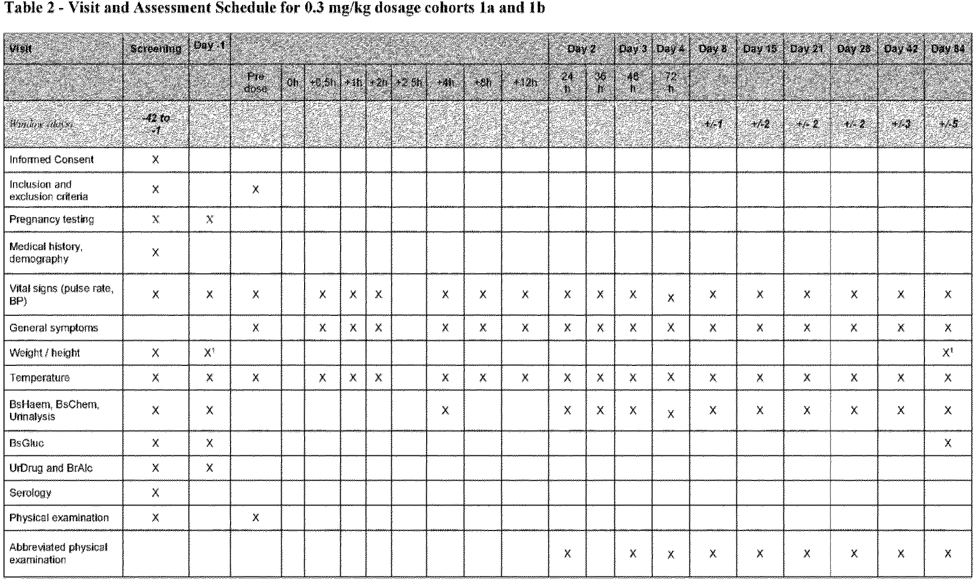

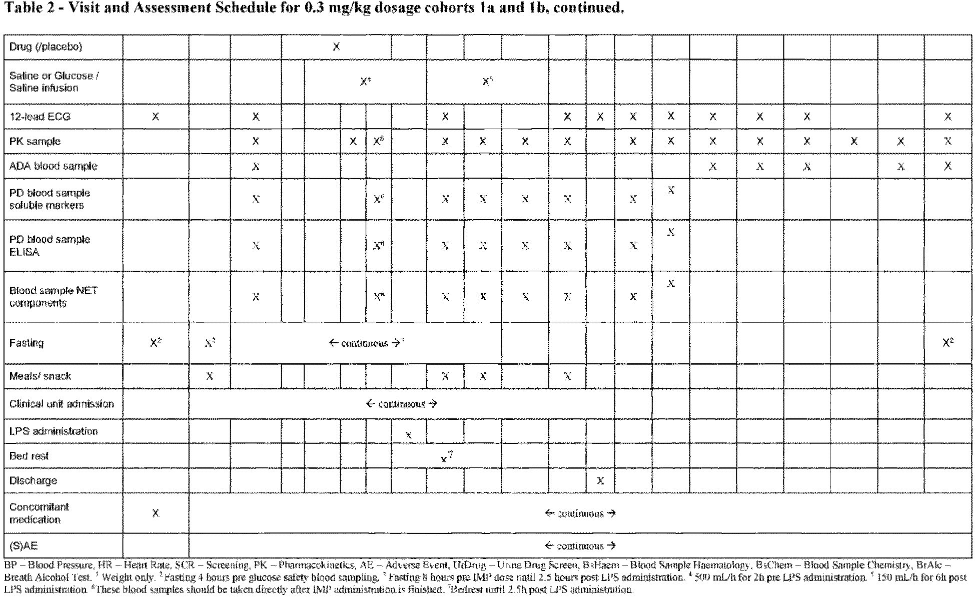

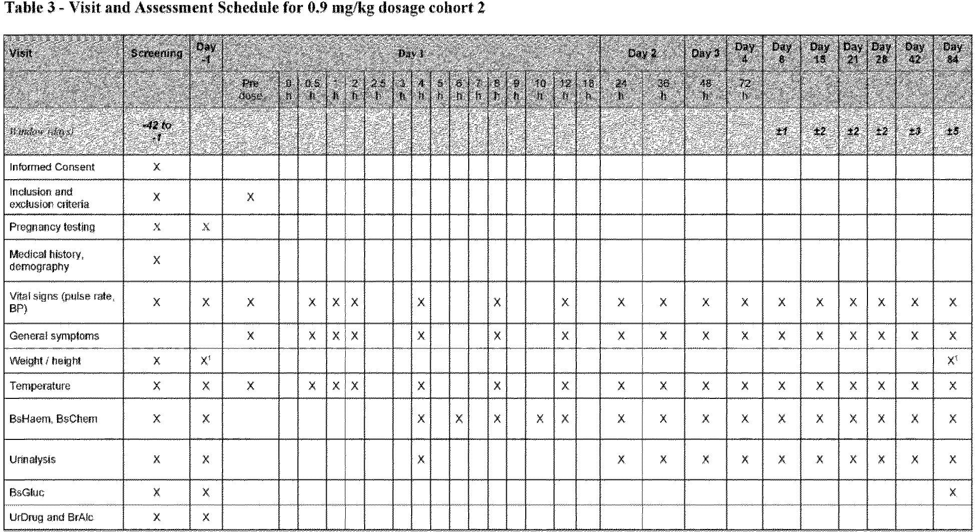

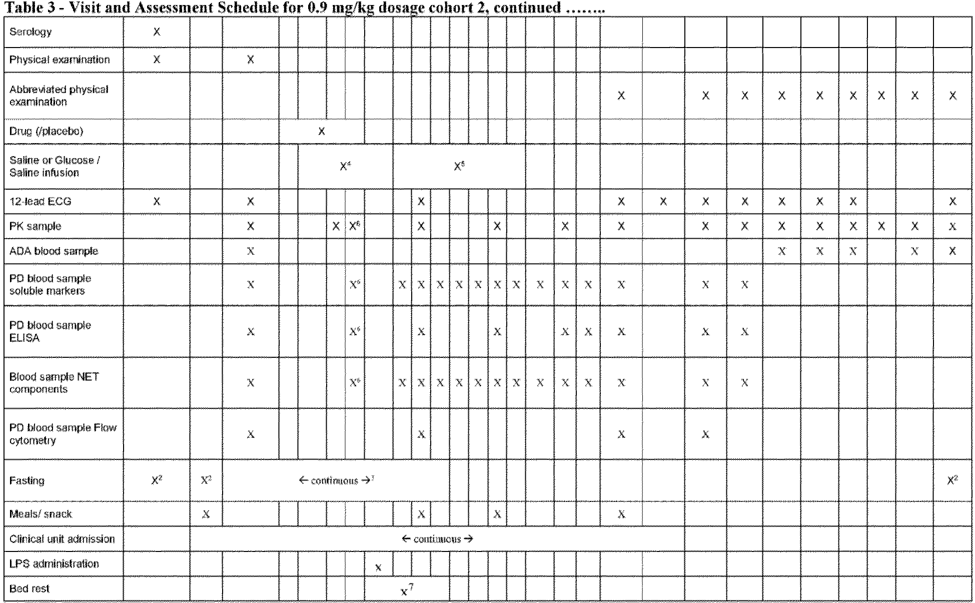

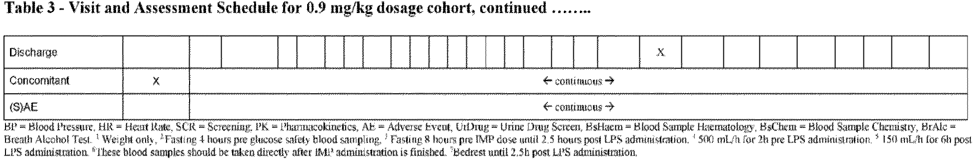

### Results

Key results in the study are shown in Figures 1 to 8. The results shown in each are depicted below.

**Figure 1** shows results for CIT-013 epitope levels (Units/ml) in serum from LPS challenged healthy volunteers treated with placebo. The measurement was only performed in the placebo groups as the assay relied on an antibody binding the epitope for CIT-013 which would cross-compete with CIT-013 present in the serum of the volunteers dosed with CIT-013. Panel A) shows the results for each placebo subject in cohort 1 (n=3). Panel B) shows the results for each placebo subject in cohort 2 (n=6).

**Figure 2** shows results for citrullinated histone 3 levels (ng/ml) in serum for cohorts of LPS challenged healthy volunteers treated with either placebo or CIT-013. LPS was dosed at 2 ng/kg two hours after placebo or CIT-013 treatment. Results for Cohort 1 are shown in Panels A) and B). Panel A) shows results for those subjects in Cohort 1 treated with placebo (n=3). Panel B) shows results for those subjects treated with 0.3 mg/kg CIT-013 (n=3). The results for Cohort 2 are shown in Panels C) and D). Panel C) shows results for Cohort 2 subjects treated with placebo (n=6). Panel D) shows results for Cohort 2 treated with 0.9 mg/kg CIT-013 (n=8). Panel E) shows the calculated area under the curve for cohort 2 placebo and CIT-013 treated healthy volunteers. A non-paired Mann-Whitney test was performed to calculate statistical differences. ***, p=0.00075.

**Figure 3** shows results for CXCL10 levels (pg/ml) measured in plasma from the two cohorts of LPS challenged healthy volunteers treated with either placebo or CIT-013. LPS was dosed at 2 ng/kg two hours after placebo or CIT-013 treatment. Cohort 1 was treated with A) placebo (n=3) or B) 0.3 mg/kg CIT-013 (n=3). Cohort 2 was treated with C) placebo (n=6) or D) 0.9 mg/kg CIT-013 (n=8). The circled datapoints at 20000 pg/ml indicate that the upper limit of quantification has been reached and that further dilution of the sample was not possible.

**Figure 4** shows results for TNF-alpha levels (pg/ml) in plasma from the two cohorts of LPS challenged healthy volunteers treated with either placebo or CIT-013. LPS was that dosed at 2 ng/kg two hours after placebo or CIT-013 treatment. Cohort 1 has been treated with A) placebo (n=3) or B) 0.3 mg/kg CIT-013 (n=3). Cohort 2 has been treated with C) placebo (n=6) or D) 0.9 mg/kg CIT-013 (n=8). Circled datapoints indicate that the upper limit of quantification has been reached and that further dilution of the sample was not possible.

**Figure 5** shows results for VCAM-1 levels (pg/ml) in plasma from the two cohorts of LPS challenged healthy volunteers treated with either placebo or CIT-013. LPS was dosed at 2 ng/kg two hours after placebo or CIT-013 treatment. Cohort 1 has been treated with A) placebo (n=3) or B) 0.3 mg/kg CIT-013 (n=3). Cohort 2 has been treated with C) placebo (n=6) or D) 0.9 mg/kg CIT-013 (n=8).

**Figure 6** shows results for IL-10 levels (pg/ml) in plasma from the two cohorts of LPS challenged healthy volunteers treated with either placebo or CIT-013. LPS was dosed at 2 ng/kg two hours after placebo or CIT-013 treatment. Cohort 1 has been treated with A) placebo (n=3) or B) 0.3 mg/kg CIT-013 (n=3). Cohort 2 has been treated with C) placebo (n=6) or D) 0.9 mg/kg CIT-013 (n=8).

**Figure 7** shows results for calprotectin levels (ng/ml) in plasma from the two cohorts of LPS challenged healthy volunteers treated with either placebo or CIT-013. LPS was dosed at 2 ng/kg two hours after placebo or CIT-013 treatment. Cohort 1 has been treated with A) placebo (n=3) or B) 0.3 mg/kg CIT-013 (n=3). Cohort 2 has been treated with C) placebo (n=6) or D) 0.9 mg/kg CIT-013 (n=8).

**Figure 8** shows results for P-selectin levels (ng/ml) in plasma from the two cohorts of LPS challenged healthy volunteers treated with either placebo or CIT-013. LPS was dosed at 2 ng/kg two hours after placebo or CIT-013 treatment. Cohort 1 has been treated with A) placebo (n=2) or B) 0.3 mg/kg CIT-013 (n=3). Cohort 2 has been treated with C) placebo (n=6) or D) 0.9 mg/kg CIT-013 (n=8).

### Example 2

Given the particular efficacy of the 0.3 mg/kg and distinctive cytokine pattern in comparison to the 0.9 mg/kg which was also effective, but without the different cytokine pattern, a lower-end point was calculated based on target engagement calculations. In particular, the calculation was based on the number of CIT-013 molecule to (fully) bind the citrullinated target epitopes on histones H2A and H4 (i.e.Target Engagement) in the blood compartment of SLE patients and LPS challenged healthy volunteers (HVs) based on the CitH3 levels measured in serum of active SLE patients which is similar or slightly lower to the levels in LPS challenged HVs. All calculations were based on the CitH3 levels measured in serum of active SLE patients which is similar or slightly lower to the levels in LPS challenged HVs.

The approach adopted was as follows:
- Levels of citrullinated H3 in serum from SLE patients were determined using an ELISA (Cayman).
- Average levels of 12 ng/mL and 19 ng/mL were found in SLE patients with active or remission disease state, respectively.
- In total circulation (5 ltr) 60 - 95 µg is present, which corresponds to 4 nmol - 6.33 nmol, assuming the estimate size of Histone H3 is 15kD.
- The findings served as a starting point to perform Target-Engagement calculations of CIT-013.

In LPS challenged HVs the volunteer having the highest CitH3 level reached approx 15 ng/ml. Which is similar to the mean CitH3 levels in SLE patients. Results obtained are shown in the Table 4 below.

**Table 4**

| | **Prism fit (4PL)** | | | |
|---|---|---|---|---|
| Subject ID | **Prism (4-parameter fit, x = conc) calculated CitH3 cone (ng/mL)** | **Cone. CitH3 (ng/mL) in undiluted sample** | **SLE active or remission** | **AVERAGE** |
| UMCG-16 | 3,117 | 31,2 | active | 12,0 |
| UMCG-16 | 5,667 | 28,3 | | |
| UMCG-11 | 0,151 | 1,5 | active | |
| UMCG-11 | 0,356 | 1,8 | | |
| UMCG-4 | 0,459 | 4,6 | active | |
| UMCG-4 | 0,947 | 4,7 | | |
| *UMCG-22* | *0,045* | *0,4* | *active* | |
| *UMCG-22* | | *0,0* | | |
| UMCG-36 | 1,093 | 10,9 | remission | 19,3 |
| UMCG-36 | 2,121 | 10,6 | | |
| UMCG-33 | 4,109 | 41,1 | remission | |
| UMCG-33 | 6,934 | 34,7 | | |
| UMCG-26 | 0,947 | 9,5 | remission | |
| UMCG-26 | 1,828 | 9,1 | | |

| | | | | |
|---|---|---|---|---|
| **values in italics were below the ELISAs limit of detection and thus have not been used to calculate the average.* | | | | |

- The following was then factored in:
- The protein size of the four core histones ranges between 11.4 kD (H4) and 15.4 kD (H3).
- Total molecular weight of the nucleosome (8-mer) is approx 110 kD.
- Histone H2A has a MW of ~14 kD (also when citrullinated).
- Histone H3 has a MW of ~15 kD (also when citrullinated).
- CIT-013 recognizes citrulline residues on histone H2A and H4, which means that per nucleosome 4 histones H2A and H4 deliver a total of 4 citrullinated epitopes.
- Ratio histones H3 : H2A/H4 = 2:4 (1:2).

The following was then taken into consideration and calculated:
1. CitH3 content in SLE or LPS challenged HV serum is approx. 15 ng/mL, which is 75 µg in 5 L blood.
2. Histone ratio H2A/H4 : H3 is 2:1, which leads to approx 150 µg CitH2A/CitH4 in 5 L blood.
3. Molecular Weight of tetrameric H2A/ or H4 is approx 15 kD so 150 µg/15000 = 10 nmol.
4. Epitope molecules present: 10 nmol x 6.03E23 = 6,022E15.
5. If CIT-013 binds simultaneously (bivalent) citrulline residues of H2A and H4, then 6,022E15 molucules need to be blocked. 6,022 molecules (10 nmol)/2 of CIT-013 are needed which corresponds to 750 µg drug.
6. If CIT-013 binds citH2A and citH4 separately (monovalent), then double amount of CIT-013 is needed: 6,022 molecules (10 nmol) corresponds to 1500 µg drug.

The calculation assumed that all citrulline residues are present and accessible for CIT-013. The calculations are based on NETs in plasma. NETs in tissue are not included

According to calculations a dose of 750 µg (based on bivalent binding) to 1500 µg (based on monovalent binding) would already suffice to bind all citrullined H2A/H4 histones in the blood compartment of SLE patients or LPS challenged HVs. That corresponds to a dose of 0.01 mg/kg to 0.02 mg/kg as the lower end of the effective range for dosage.

### Example 3: Determine the presence of CIT-013' epitope in Rheumatoid Arthritis serum samples

### Overview

The presence of CIT-013's epitope(s) in serum from Rheumatoid Arthritis (RA) patients and compared this with epitope levels in serum from healthy volunteers. By using the in-house developed epitope ELISA, it was clearly demonstrated that Neutrophilic Extracellular Traps (NETs) containing citrullinated histones H2A and H4 could be detected in RA serum and that the level of NETs was higher compared to serum from healthy volunteers. These findings further show the potential of CIT-013 for the treatment of Rheumatoid Arthritis patients. The main objective of this study was to determine the presence of CIT-013's epitope in serum from Rheumatoid Arthritis patients and to investigate if these levels were enhanced in the RA population when compared to serum from healthy subjects.

### Materials & Methods

### Human serum samples

A well characterized serum cohort of 58 Rheumatoid Arthritis (RA) patients which gave informed consent was received from the -80° C repository of the Biobank from the Radboud University Medical Centre at Nijmegen (The Netherlands) on 23rd December 2021. Upon receipt at Citryll the samples were aliquoted and stored in a temperature-controlled freezer at -80° C, until analysis.

Normal human serum from 35 healthy subjects was obtained via venipuncture in serum separator tubes (SST), and processed to serum, aliquoted and stored at -80° C. All healthy subjects provided inform consent and were recruited from different companies located at the Pivot Park at Oss (The Netherlands). Blood processing was performed at Citryll using standard procedure (allow clotting 30 - 45' at RT, spin down at 1,300 g and aliquot serum) The serum samples from both RA patients and healthy volunteers (HV) were analyzed using the in-house developed 'epitope' ELISA. This ELISA determines the presence of citrullinated histone H2A and H4 in the NET structure, and as such determines CIT-013's epitopes. Critical reagents for ELISA execution are listed in table 5 below.

**Table 5: critical reagents for the 'epitope' ELISA**

| **Item** | **Supplier** | **Cat. #** | **Batch #** |
|---|---|---|---|
| D-PBS (1x) | Gibco | 14190-144 | 2333792 |
| PBS, 20X liquid concentrate, Ultra Pure Grade | *VWR* | E703-1L | 2061956779 |
| Tween 20 (Polysorbate) | *VWR* | 663684B | 18C084001 |
| Recombinant Anti-Histone H3 (citrulline R2+R8+R17) antibody | Abeam | Ab281584 | GR3389586-12 |
| ELK skimmed milk powder | Friesland Campina | NA | L112359 09:17 |
| Polyclonal Goat Anti Mouse Immunoglobulin HRP | Dako | P0447 | 20083051 |
| Mouse ACHA (previously called mtACPA) | Citryll | MQ22.101 | 210017 |
| TMB stabilized Chromogen | Invitrogen | SB02 | 77197935 |
| Sulfuric acid (95-97%) | Merck | 1007311000 | K51658331931 |
| NUNC Maxisorp plates | Thermo Scientific | 442404 | |

### Data and statistical analysis

The data obtained from the epitope ELISA demonstrated elevated epitope levels in RA cohort when compared to HV cohort. To demonstrated significance a Mann-Whitney test (unpaired; nonparametric; two tailed P-value and 95% confidence level) was executed using GraphPad Prism software (GraphPad Prism, San Diego, USA). A significant difference between RA cohort and HV cohort was considered when the p-value was <0.05. With a p-value of 0.074 no significant difference was established.

### NET Detection

The method for detection of circulating NETs in serum samples from both RA patients and healthy subjects was the in house developed 'epitope' (i.e., citrullinated histone H2A and H4) ELISA (Fig 10). The serum samples, together with a dilutional series of activated NETs as standard, as well as quality controls were pipetted into the wells of a coated microtiter plate. The coating was a recombinant multiclonal antibody specific for citrullinated histone H3. Any NET containing citrullinated histone H3 is bound by the immobilized antibody and after washing away unbound substances, a mouse anti-citrullinated histone antibody (ACHA) specific for the citrullinated histone H2A and H4 was added to the wells. Following a wash to remove any unbound mouse ACHA, an enzyme-linked polyclonal Goat anti-mouse IgG antibody was added to the wells. Following a final wash to remove any unbound antibody-enzyme reagent, a substrate solution (TMB) was added to the wells and colour developed in proportion to the amount of citrullinated NETs present in the wells. The colour development was stopped using a strong acid and the optical density was measured (at wavelength 450, 620 nm) using a Tecan ELISA reader. All calibrators and quality controls were prepared freshly on the day of analysis. The serum samples were diluted five times on day of analysis. Each calibrator, quality controls and serum samples were measured in duplicate (i.e. two wells). The coefficient of variation (%CV) of each duplicate measurement had to be ≤ 30.0% (calculated as: CV% = 100 * SD of the duplicate measurement / mean of the duplicate measurement)

Samples were analyzed using a 96-well absorption reader (Infinite F50 Plus; Tecan Ltd, Männedorf, Switzerland). Data processing was performed using GraphPad Prism (version 9.1.0) software supplied by GraphPad Prism Software (San Diego, USA). Sample concentrations were calculated using a 4 Parameter Logistic (4-PL) nonlinear regression model; no weighting factor.

The LLOQ was 0.625 U/mL and the ULOQ was 120 U/mL for Epitope (i.e., citrullinated NETs) in 100% serum of both RA and HV.

### Results & Discussion

The serum samples from both RA patients and healthy volunteers were analyzed in the epitope ELISA in one analytical run and on three different plates. The calibration curves were comparable as demonstrated in Figure 10a and the precision (%CV) and recovery (%RE) were between 2.9 and 20.7%, and 92.3 and 107.9%, respectively (Table 2). The %CV of the quality controls were between 1.3 and 9.8%, the %RE was between 132 and 277%. Although the recoveries of the QCs were too high, it was decided to report the obtained NET data from the RA and HV serum samples, as these were relatively compared to each other and the calibration curves for all three plates were very comparable (Fig 1a).

The back-calculated values of all the analyzed serum samples were determined against the calibration curve and plotted (Figure 10b). Here we demonstrated that the epitope levels in serum from the RA cohort were higher compared to serum from the HV cohort. Although there is an inequality in group size it was clear that the level of citrullinated NETs in RA patients was higher compared to healthy subjects.

**Table 6: Precision (%CV) and Accuracy (%RE) of the calibrators**

| | **Concentration NETs (U/mL)** | | | | | | | | | | | |
|---|---|---|---|---|---|---|---|---|---|---|---|---|
| | **24** | **20** | **16** | **12** | **8** | **4** | **2** | **1** | **0,5** | **0,25** | **0,125** | **0,0625** |
| Plate #1 | 20,57 | 16,20 | **14,59** | 10,31 | **7,08** | 3,39 | **1,88** | 0,93 | 0,54 | 0,26 | 0,11 | 0,06 |
| | 27,46 | 24,25 | 18,30 | 14,77 | 9,20 | 4,44 | 2,20 | 0,99 | 0,55 | 0,25 | 0,12 | 0,05 |
| Plate #2 | 20,34 | 16,53 | 12,83 | 10,97 | 5,37' | 3,61 | 2,05 | **0,96** | 0,52 | 0,23 | 0,12 | 0,07 |
| | 27,91 | 23,53 | 20,49 | 15,79 | 9,29 | 4,59 | 2,24 | 0,92 | 0,53 | 0,23 | 0,12 | 0,07 |
| Plate #3 | 26,15 | 18,81 | 13,97 | 12,59 | 7,45 | 3,67 | 2,12 | 0,91 | 0,56 | 0,26 | 0,13 | 0,05 |
| | 23,08 | 21,14 | 15,82 | 12.90 | 8,95 | 3,97 | 2,07 | 0.95 | 0,53 | 0.25 | 0,12 | 0,04 |
| Mean | 24,25 | 20,08 | 16,00 | 12,89 | 8,39 | 3,95 | 2,09 | D,94 | 0,54 | 0,25 | 0,12 | 0,06 |
| StDEV | 3,39 | 3,46 | 2,88 | 2,12 | 1,05 | 0,48 | 0,13 | 0,03 | 0,02 | 0,02 | 0,01 | 0,01 |
| %CV | 14,0 | 17,2 | 18,0 | 16,4 | 12,5 | 12,1 | 6,1 | 2,9 | 2,9 | 6,2 | 5,1 | 20,7 |
| %RE | 101,0 | 100,4 | 100,0 | 107,4 | 104,9 | 98,7 | 104,6 | 94,3 | 107,9 | 99,1 | 95,6 | 92,3 |
| n | 6 | 6 | 6 | 6 | 5 | 6 | 6 | 6 | 6 | 6 | 6 | 6 |

The presence of citrullinated NETs in serum from RA patients opens the possibility for treatment of Rheumatoid Arthritis with the first in class therapeutic drug CIT-013. The observation that the NET content in RA patients is elevated relative to the NET content in HV is in line with Bach et al. (2020) Arthritis & Rheumatology; 72: pp 47-56. In their study, it was demonstrated that RA patients in three different cohorts (2 plasma; 1 serum) had markedly elevated NET levels compared to healthy controls irrespective of the matrix used (p value of <0.001 in all three cohorts). Bach et al. determined the NET content in plasma and serum matrix by analysis of myeloperoxidase:DNA complexes, whilst in our study specifically citrullinated NETs containing CIT-013's epitopes were measured using the in house developed epitope ELISA.

### Conclusions

In serum from RA patients, the citrullinated NET and epitope content was higher in comparison to serum from HVs. Since NETs are contributing to RA disease development and pathology, these findings provide support for the development of CIT-013 for the treatment of RA patients. Based on this study it would be possible so set the threshold for epitope levels for the recruitable RA population for a CIT-013 clinical study at for example > 10 U/mL.

### Appendix: List of abbreviations

%CV percentage coefficient of variation
%RE percentage recovery
DNA Deoxyribonucleic Acid
ELISA Enzyme-Linked Immunosorbent Assay
HV Healthy Volunteers
ACHA Anti Citrullinated Histone Antibody
NET Neutrophil Extracellular Trap
PAD4 peptidylarginine deiminase 4
RA Rheumatoid Arthritis
RT Room Temperature
SST Serum Separation Tubes
TMB 3,3',5,5'-Tetramethylbenzidine
U/mL Units per milliliter

### Sequence listing

SEQ ID NO: 1- CDR1 of msVH22.101 and hVH22.101(HC)x
   GYTFTNYG
SEQ ID NO: 2- CDR2 of msVH22.101 and hVH22.101(HC)x
   INTYSGEA
SEQ ID NO: 3- CDR3 of msVH22.101 and hVH22.101(HC)x
   LRGYTYQSFDEGGDY
SEQ ID NO: 4- CDR2 of msVL22.101 and hVL22.101(LC)y
   LVS
SEQ ID NO: 5- CDR3 of msVL22.101 and hVL22.101(LC)y
   WQGTHFPYT
SEQ ID NO: 6- CDR1 of hVL22.101LC17
   QSLLDTDGKTY
SEQ ID NO: 7- CDR1 of hVL22.101LC21
   QSLLDSDAKTY
SEQ ID NO: 8- CDR1 of hVL22.101LC27
   QSLLDTDAKTY
SEQ ID NO: 9- CDR1 of hVL22.101LC41
   QSLLDADGKTY
SEQ ID NO: 10- CDR1 of hVL22.101LC42
   QSLLDNDGKTY
SEQ ID NO: 11- hVH22.101f 
RIQLVQSGAEVKKPGASVKVSCKASGYTFTNYGMHWVRQAPGQGLEWMGWINTYSGEATYAQKFQGRVTMTRDTSISTAYM
ELSRLRSDDTAVYYCLRGYTYQSFDEGGDYWGQGTLVTVSS
SEQ ID NO: 12- hVH22.101HC9 
RIQLVQSGAEVKKPGASVKVSCKASGYTFTNYGMHWVRQAPGQGLEWMGWINTYSGEATYVDDFQGRVTMTRDTSISTAYM
ELSRLRSDDTAVYYCLRGYTYQSFDEGGDYWGQGTLVTVSS
SEQ ID NO: 13- hVL22.101LC17 
DVVMTQSPLSLPVTLGQPASISCRSSQSLLDTDGKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 14- hVL22.101LC21 
DVVMTQSPLSLPVTLGQPASISCRSSQSLLDSDAKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 15- hVL22.101LC27 
DVVMTQSPLSLPVTLGQPASISCRSSQSLLDTDAKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 16- hVL22.101LC41 
DVVMTQSPLSLPVTLGQPASISCRSSQSLLDADGKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 17- hVL22.101LC42 
DVVMTQSPLSLPVTLGQPASISCRSSQSLLDNDGKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 18- SEQ ID NO 1 from WO2016092082 (used in Example 117) from histone 2A
   SGXGKQGGKARA
   Where X is citrulline
SEQ ID NO: 19- SEQ ID NO 2 from WO2016092082, (used in Example 7) from histone 4
   SGXGKGGKGLGKGGAKRHRKVLR
   Where X is citrulline
SEQ ID NO: 20- Shortened SEQ ID NO 2 from WO2016092082 (used in Example 7) from histone 4
   SGXGKGGKGLGK
   Where X is citrulline
SEQ ID NO: 21- Peptide no 4 (human histone 2A) (SEQ ID NO 24 from WO2011070172)
   QFPVGXVHRLLR
   Where X is citrulline
SEQ ID NO: 22- Peptide no 6 (human histone 2A) (SEQ ID NO 26 from WO2011070172)
   VHRLLXKGNYSE
   Where X is citrulline
SEO ID NO: 23- Human heavy chain constant domain of IgGl 
ASTKGPSVFPLAPSSKSTSGGTAALGCLVKDYFPEPVTVSWNSGALTSGVHTFPAVLQSSGLYSLSSVVTVPSSSLGTQTY
ICNVNHKPSNTKVDKKVEPKSCDKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSKLTVDKSRWQQGNVFSCSVMHEALHNHYTQKSL
SLSPG
SEQ ID NO: 24- Human kappa chain constant domain
SEQ ID NO: 25- msVH22.101 
RIQLVQSGPELKKPGEAVKISCKASGYTFTNYGMHWMKQTPGKDFRWMGWINTYSGEATYVDDFKGRFAFSLGTSASTAYL
QINNLKNDDTATYFCLRGYTYQSFDEGGDYWGQGTALTVSS
SEQ ID NO: 26- hVH22.101j 
QVQLVQSGAEVKKPGASVKVSCKASGYTFTNYGMHWVRQAPGQGLEWMGWINTYSGEATYAQKFQGRVTMTRDTSISTAYM
ELSRLRSDDTAVYYCLRGYTYQSFDEGGDYWGQGTLVTVSS
SEQ ID NO: 27- hVH22.101HC7 
QVQLVQSGAEVKKPGSSVKVSCKASGYTFTNYGMHWVRQAPGQGLEWMGWINTYSGEATYAQKFQGRVTITADESTSTAYM
ELSSLRSEDTAVYYCLRGYTYQSFDEGGDYWGQGTLVTVSS
SEQ ID NO: 28- hVH22.101HC8 
QVQLVQSGAEVKKPGSSVKVSCKASGYTFTNYGMHWVRQAPGQGLEWMGWINTYSGEATYVDDFQGRVTITADESTSTAYM
ELSSLRSEDTAVYYCLRGYTYQSFDEGGDYWGQGTLVTVSS
SEQ ID NO: 29- hVH22.101HC10 
QVQLVQSGAEVKKPGASVKVSCKASGYTFTNYGMHWVRQAPGQGLEWMGWINTYSGEATYVDDFQGRVTMTRDTSISTAYM
ELSRLRSDDTAVYYCLRGYTYQSFDEGGDYWGQGTLVTVSS
SEQ ID NO: 30- msVL22.101 
DVVMTQTPLTLSVTTGQPASISCKSSQSLLDSDGKTYLNWLFQRPGQSPKRLIYLVSKLDSGVPDRFTGSGSGTDFTLKIS
RVEAEDLGIYYCWQGTHFPYTFGGGTNLEIK
SEQ ID NO: 31- hVL22.101e 
DVVMTQSPLSLPVTLGQPASISCRSSQSLVDSDGKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 32- hVL22.101g 
DVVMTQSPLSLPVTLGQPASISCRSSQSLLDSDGKTYLNWFQQRPGQSPRRLIYLVSKLDSGVPDRFSGSGSGTDFTLKIS
RVEAEDVGVYYCWQGTHFPYTFGQGTKLEIK
SEO ID NO: 33- hVL22.101h 
DVVMTQSPLSLPVTLGQPASISCRSSQSLVASDGKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 34- hVL22.101i 
DVVMTQSPLSLPVTLGQPASISCRSSQSLVESDGKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 35- hVL22.101j 
DVVMTQSPLSLPVTLGQPASISCRSSQSLVSSDGKTYLNWFQQRPGQSPRRLIYLVSKLDSGVPDRFSGSGSGTDFTLKIS
RVEAEDVGVYYCWQGTHFPYTFGQGTKLEIK
SEQ ID NO: 36- CDR1 of msVL22.101 and hVL22.101g
   QSLLDSDGKTY
SEQ ID NO: 37- CDR1 of hVL22.101e
   QSLVDSDGKTY
SEQ ID NO: 38- CDR1 of hVL22.101h
   QSLVASDGKTY
SEQ ID NO: 39- CDR1 of hVL22.101i
   QSLVESDGKTY
SEQ ID NO: 40- CDR1 of hVL22.101j
   QSLVSSDGKTY
SEQ ID NO: 41- CDR1 of hVL22.101LC16
   QSLLESDGKTY
SEQ ID NO: 42- CDR1 of hVL22.101LC19
   QSLLDSEGKTY
SEQ ID NO: 43- CDR1 of hVL22.101LC20
   QSLLDSSGKTY
SEQ ID NO: 44- CDR1 of hVL22.101LC22
   QSLLESEGKTY
SEQ ID NO: 45- CDR1 of hVL22.101LC23
   QSLLESSGKTY
SEQ ID NO: 46- CDR1 of hVL22.101LC24
   QSLLESDAKTY
SEQ ID NO: 47- CDR1 of hVL22.101LC25
   QSLLDTEGKTY
SEQ ID NO: 48- CDR1 of hVL22.101LC26
   QSLLDTSGKTY
SEQ ID NO: 49- CDR1 of hVL22.101LC37
   QSLLDSAGKTY
SEQ ID NO: 50- CDR1 of hVL22.101LC38
   QSLLESAGKTY
SEQ ID NO: 51- CDR1 of hVL22.101LC39
   QSLLDAEGKTY
SEQ ID NO: 52- CDR1 of hVL22.101LC40
   QSLLDNEGKTY
SEQ ID NO: 53- msFibβ XG (SEQ ID NO 37 from WO2011070172)
   EPTDSLDAXGHRPVDRR
   Where X is citrulline
SEQ ID NO: 54- msVim XS/XL (SEQ ID NO 38 from WO2011070172)
   YVTXSSAVXLXSSVP
   Where X is citrulline
SEQ ID NO: 55- Region around CDR2 of msVL22.101 and hVL22.101(LC)y
   LVSKLDS
SEQ ID NO: 56- Heavy chain constant domain of hCH22.101f

## Claims

1. An antibody or binding fragment thereof that specifically binds to a citrullinated epitope on deiminated human histone 2A and/or histone 4 for use in a method of treating or preventing a disease associated with extracellular trap release from cells, such as a Neutrophil Extracellular Trap (NET)-associated pathology (NET associated pathology) or an Eosinophil Extracellular Trap (EET) -associated pathology (EET-associated pathology) in a subject in need thereof, wherein the method comprises administering at least one dose of the antibody or binding fragment thereof at a dosage of from about 0.01 mg/kg to about 0.6 mg/kg.

2. The antibody or binding fragment thereof for the use of claim 1, wherein the antibody or binding fragment thereof is administered at a dosage of:
(a) from about 0.02 mg/kg to about 0.5 mg/kg;
(b) from about 0.05 mg/kg to about.0.4 mg/kg; or
(c) from about 0.1 mg/kg to about 0.3 mg/kg

3. The antibody or binding fragment thereof for the use of claim 1, wherein the antibody or binding fragment thereof is administered at a dosage of about 0.3 mg/kg.

4. The antibody or binding fragment thereof for the use of any one of the preceding claims, wherein:
(a) the antibody or binding fragment thereof is administered to the subject intravenously or subcutaneously; and/or
(b) the administration of the antibody or binding fragment thereof increases the level of IL-10 in the subject; and/or
(c) the administration of the antibody or binding fragment thereof suppresses the level of at least one of calprotectin, P-selectin, CXCL10, TNF-alpha, and VCAM in the subj ect.

5. The antibody or binding fragment thereof for the use of any one of the preceding claims, wherein:
(a) 1 to 10 doses of the antibody are administered;
(b) 2 to 8 doses of the antibody are administered; or
(c) 4 to 6 doses of the antibody or binding fragment are administered, optionally wherein a plurality of doses are administered at intervals of:
(i) 1 week to 3 months;
(ii) 1 week to 3 weeks; or
(iii) about 2 weeks.

6. The antibody or binding fragment thereof for the use of any one of the preceding claims, wherein the antibody or binding fragment thereof comprises:
a) CDR1 of VL, wherein the CDR comprises or consists of the amino acid sequence QSL-X₁-D-X₂-D-X₃-KTY, wherein X₁ is V or L, X₂ is T, S, A or N and X₃ is G or A, provided that the amino acid sequence is not QSLLDSDGKTY (SEQ ID NO: 36) or QSLVDSDGKTY (SEQ ID NO: 37); and
b) at least one CDR selected from SEQ ID NOs: 1 to 5.

7. The antibody or binding fragment thereof for the use of claim 6, wherein the antibody or binding fragment thereof comprises:
a) the VL CDR 1 of SEQ ID NO: 9 (QSLLDADGKTY); and
b) the VH CDR 1 of SEQ ID NO: 1 (GYTFTNYG), the VH CDR 2 of SEQ ID NO: 2 (INTYSGEA), the VH CDR 3 of SEQ ID NO: 3 (LRGYTYQS FDEGGDY), the VL CDR 2 of SEQ ID NO: 4 (LVS) and the VL CDR 3 of SEQ ID NO: 5 (WQGTHFPYT), optionally wherein the antibody or binding fragment thereof comprises:
i) VL CDR1 of SEQ ID NO: 9;
ii) VL CDR2 of SEQ ID NO:4 and VL CDR3 of SEQ ID NO:5; and
iii) the heavy chain variable domain amino acid sequence of SEQ ID NO: 11 or 12.

8. The antibody or binding fragment thereof for use of claim 6 or claim 7, wherein the antibody or binding fragment thereof comprises:
a) the heavy chain variable domain amino acid sequence set forth in SEQ ID NO: 11 and the light chain variable domain amino acid sequence set forth in SEQ ID NO: 16;
or
b) the heavy chain variable domain amino acid sequence set forth in SEQ ID NO: 12 and the light chain variable domain amino acid sequence set forth in SEQ ID NO: 16.

9. The antibody or binding fragment thereof for the use of any one of the preceding claims, wherein the NET-associated pathology is a tauopathy, systemic lupus erythematosus (SLE), lupus, sepsis, vasculitis, inflammatory arthritis, rheumatoid arthritis and osteoarthritis, psoriasis, hidradenitis suppurativa, Alzheimer's disease, autoimmune hepatitis, juvenile idiopathic arthritis, myositis (polymyositis and dermatomyositis), Sjögren's disease, Anti-phospholipid Syndrome, Bechet's disease, spondylitis, spondyloarthropathy, multiple system atrophy, Parkinson's disease, Lewy body dementia asthma, allergic rhinovirus exacerbated asthma, allergic asthma, acute respiratory distress syndrome, cystic fibrosis, fibrosis and idiopathic pulmonary fibrosis, heart failure, atherosclerosis, dry eye disease, uveitis, nongranulomatous uveitis, granulomatous uveitis, dermatitis, atopic dermatitis, COPD, bronchitis, thrombotic diseases, cardiovascular diseases or another NET-associated pathology such as wound healing in diabetes, cancer, cancer metastasis, and transplant organ health *in vivo* or *ex vivo.*

10. The antibody or binding fragment thereof for the use of any one of the preceding claims, wherein the NET-associated pathology is Parkinson's disease.

11. The antibody or binding fragment thereof for the use of any one of claims 1 to 8, wherein the EET-associated pathology is an eosinophilic disease or condition, such as eosinophilic disease or condition of the skin; a respiratory eosinophilic disease or condition; a gastro-intestinal eosinophilic disease or condition; an allergic disease or condition; or a helminth, fungal, viral, or bacterial infection; or an eosinophilic disease or condition disease or condition such as Bullous Pemphigoid, Atopic dermatitis, allergic contact dermatitis, Eosinophilic Asthma, Chronic RhinoSinusitis with Nasal Polyposis (CRSwNP), Allergic sinusitis, Allergic bronchopulmonary aspergillosis, Eosinophilic chronic rhinosinusitis, Eosinophilic Esophagitis (EoE), HyperEosinophilic Syndrome (HES), Eosinophilic Granulomatosis with PolyAngitis (EGPA), Eosinophilic otitis media (EOM), and Drug Reaction with Eosinophilic & Systemic Symptoms (DRESS), arteriosclerosis, and vasculitis.

12. A method for treating a Neutrophil Extracellular Trap associated pathology (a NET-associated pathology) in a subject in need thereof, the method comprising administering to the subject at least one dose an antibody or binding fragment thereof that specifically binds to a citrullinated epitope on deiminated human histone 2A and/or histone 4 to the subject, wherein each dose of the antibody or binding fragment thereof is at a dosage of from about 0.01 mg/kg to about 0.6 mg/kg.

13. The method of claim 12, wherein:
(a) the dosage of the antibody or binding fragment thereof administered is as defined in claim 2 or 3;
(b) the route of administration of the antibody or binding fragment thereof is as defined in claim 4;
(c) the administration of the antibody or binding fragment thereof increases the level of IL-10 in the subject;
(d) the administration of the antibody or binding fragment thereof suppresses the level of at least one of calprotectin, P-selectin, CXCL10, TNF-alpha, and VCAM in the subject;
(e) the sequence of the antibody is as defined in any one of claims 6 to 8; and/or
(f) the NET-associated pathology is as defined in claim 9 or claim 10.

14. A method for treating an Eosinophil Extracellular Trap (EET) -associated pathology (EET-associated pathology) in a subject in need thereof, the method comprising administering to the subject at least one dose an antibody or binding fragment thereof that specifically binds to a citrullinated epitope on deiminated human histone 2A and/or histone 4 to the subject, wherein each dose of the antibody or binding fragment thereof is at a dosage of from about 0.01 mg/kg to about 0.6 mg/kg.

15. The method of claim 14, wherein:
(a) the dosage of the antibody or binding fragment thereof administered is as defined in claim 2 or 3;
(b) the route of administration of the antibody or binding fragment thereof is as defined in claim 4;
(c) the administration of the antibody or binding fragment thereof increases the level of IL-10 in the subject;
(d) the administration of the antibody or binding fragment thereof suppresses the level of at least one of calprotectin, P-selectin, CXCL10, TNF-alpha, and VCAM in the subject;
(e) the sequence of the antibody is as defined in any one of claims 6 to 8; and/or
(f) the EET-associated pathology is as defined in claim 11.
